# EUROPEAN PATENT APPLICATION

(11) **EP 3 935 942 A1**
(43) Date of publication of application: **12.01.2022**
(21) Application number: 20184798.5
(22) Date of filing: 08.07.2020
(51) Int. Cl.: A01N 1/00, C07K 14/435, C12N 1/04

(54) **MEDIUM ADDITIVE FOR REDUCING STORAGE-ASSOCIATED DEATH OF LEUKOCYTES AND STEM CELLS**

(71) Applicant: Friedrich-Alexander-Universität Erlangen-Nürnberg, 91054 Erlangen (DE)
(72) Inventor: Hackstein, Holger, 91054 Erlangen (DE)
(74) Representative: Wichmann, Hendrik

(57) **Abstract**

The present invention relates to the use of sericin as a medium additive for reducing storage-associated death of leukocytes or stem cells stored in an aqueous storage medium at a cell density of 0.001 to 2·10⁸ cells/mL and at a temperature of up to 30°C where the storage medium is liquid. The invention further relates to a corresponding method for storing such cells for a period of at least 24 hours up to 6 weeks, to sericin-containing aqueous cell storage media containing no or only very low amounts of one or more of the vitamins, amino acids and other compounds which are usually essential for the culture of animal cells, to dry medium formulations or medium concentrates of such storage media as well as to corresponding suspensions of leukocytes or stem cells in such storage media.

## Description

### FIELD OF THE INVENTION

The present invention generally relates to the use of sericin as a medium additive for reducing storage-associated death of leukocytes or stem cells stored in an aqueous storage. The invention further relates to a corresponding method for storing such cells, to sericin-containing aqueous cell storage media containing no or only very low amounts of one or more of the vitamins, amino acids and other compounds which are usually essential for the culture of animal cells, to dry medium formulations or medium concentrates of such storage media as well as to corresponding suspensions of leukocytes or stem cells in such storage media.

### BACKGROUND OF THE INVENTION

Leukocytes und stem cells comprise a number of different cell types which vary with regard to nutrient requirement and cell metabolism. This is why a multitude of different nutrient and culture media has been developed. Preservation of viable leukocytes or stem cells (suspension cells) for longer periods of time, such as a week or longer, currently requires these cells to be transferred to DMSO-containing cryopreservation media and to be frozen at low temperatures (typically -20°C to -196°C). The downsides of this standard preservation technique include the high technical effort and the deterioration of cell viability due to freezing and thawing. Moreover, there is no scientific gold standard for the short-term storage (hours to a few days) of leukocytes or stem cells without freezing. In practice, a multitude of different culture media, additives and processes are used for short-term storage. On the other hand, in the field of transfusion medicine, nucleated suspension cells from blood are stored under GMP conditions (Good-Manufacturing Practice conditions) for further processing up to 72 hours. Successive methods for long-term stabilization and storage do not currently exists.

Consequently, there is a need for a way to store leukocytes and stem cells for a longer period of time (such as for a week or longer) that preserves cell viability without the downsides of the current standard long-term storage technique that relies on cryopreservation.

Sericin that can be extracted from the cocoons of silkworm, *Bombyx mori* has been described as an additive for cell culture media that can stimulate the growth of animal cells. Such growth-stimulating effect has also been observed with chemically synthesized sericin and sericin obtained by recombinant expression. See EP 1 302 544 B1. Four different sericin genes (*Ser1* to *Ser4*) have been described in the literature, wherein *Ser1* products have been described to make up 26 wt-%, and *Ser3* protein 3%, of the sericin proteins extracted from silkworm cocoons. See Dong et al., 2019. Uses of the cell growth-promoting effect of sericin in wound dressings and skin care products to improve wound healing and skin regeneration has been described in EP 1 335 018 A1. For the storage of differentiated (pigmented) human retinal pigment epithelium (hRPE), which can be useful as a transplant in retinal diseases such as age-related macular degeneration, sercin has been described to have a positive effect on the preservation of hRPE pigmentation on cell viability during a ten-day storage at 4°C. See Pasovic et al., 2018.

### SUMMARY OF THE INVENTION

It has now been surprisingly found that sericin can be used as a medium additive for reducing storage-associated death of leukocytes and stem cells in a liquid medium, i.e. in a non-frozen state. This allows for such cells to be stored without freezing for several days or weeks.

Compared to the current standard cryopreservation techniques, cell storage without freezing is associated with significantly less personnel expenditure, logistics effort and costs for equipment and materials.

Further, the herein described use of sericin can advantageously expand the time frame available for laboratory diagnostics of leukocytes to several days to weeks. In most cases, said time frame is presently limited to less than 24 hours due to the decrease in cell viability over time within the relevant period between taking a cell sample from the subject (e.g., the patient) and analysis of the sample.

Moreover, humane leukocyte populations such as CD3+ T-cells and CD14+ monocytes are the starting material for the preparation of innovative cell therapeutics, so called ATMPs (advanced therapy medicinal products) such, for example leukocytes expressing chimeric antigen receptors (CARs)and dendritic cell vaccines against cancer and infectious diseases. Currently, these cellular starting materials are usually obtained in blood donation centers which are often far away from each other, and then transported to the ATMP production facilities under severe time pressure due to the time-dependent decrease in cell viability being a crucial quality criterion. The herein disclosed use of sericin for reducing storage-associated death of leukocytes and stem cells allows for the cellular starting materials for ATMPs to be transported to the ATMP production facility under significantly less time pressure, which facilitates the production of high-quality ATMPs.

As demonstrated in the examples, the activity of sericin in reducing storage-associated death was observed with different media, even in the absence of serum, amino acids, vitamins and other compounds (such as insulin, transferrin and selenium) which are usually essential for the culture of animal cells.

Accordingly, the present invention relates to the use of sericin as a medium additive for reducing storage-associated death of cells selected from leukocytes and stem cells, as it is defined in the claims.

The present invention further relates to a method for storing cells selected from leukocytes and stem cells, as it is defined in the claims.

In an additional aspect, the invention relates to a medium for storing cells selected from leukocytes and stem cells. Said medium contains no or only very low amounts of one or more of the vitamins, amino acids and/or other compounds (such as insulin, transferrin and selenium) which are usually essential for the culture of animal cells, as it is defined in the claims.

Additional aspects of the invention relate to a dry medium formulation or a medium concentrate that can be reconstituted so as to form the medium of the invention, and to a suspension of cells selected from leukocytes and stem cells in the medium of the invention, as they are defined in the claims.

### DETAILED DESCRIPTION OF THE PREFERRED EMBODIMENTS

The present disclosure provides the use of sericin as a medium additive for reducing storage-associated death of cells selected from leukocytes and stem cells as well as a method for storing such cells using a sericin-containing storage medium.

### Leukocytes and stem cells

The present disclosure is applicable to leukocytes and stem cells, in particular leukocytes and stem cells from a mammal (e.g. human, monkey, mouse, rat, rabbit, dog, pig, etc.), and preferably are of human origin.

Leukocytes, also known as 'white blood cells', are nucleated cells which are produced and derived from hematopoietic stem cells, are found throughout the body including blood and lymph, and are part of the body's immune system. Leukocytes include different cell types which can be classified by cell lineage (myeloid cells or lymphoid cells) and by structure. Particular groups of leukocytes include lymphocytes (which include T lymphocytes, B lymphocytes and natural killer (NK) cells), monocytes (which are macrophage precursors), dendritic cells, and granulocytes (which include neutrophils, eosinophils, basophils and Mast cells).

In particular embodiments, the leukocytes are selected from T lymphocytes, B lymphocytes, NK cells, monocytes, dendritic cells and granulocytes. In preferred embodiments, the leukocytes are T lymphocytes or monocytes, more preferably CD3 positive T lymphocytes or CD14 positive monocytes.

Stem cells are undifferentiated or partially differentiated cells that can differentiate into various types of cells and can reproduce (self-renew) indefinitely. Stem cells include embryonic stem cells (ESCs), which are found in embryos and can be isolated and then cultured *in vivo.* Stem cells also include adult stem cells which include, but are not limited to, basal cells (which maintain the skin epithelium), mesenchymal cells (which maintain bone, cartilage, muscle and fat cells), and hematopoietic stem cells (which replenish blood and immune cells).

In particular embodiments, the stem cells are hematopoietic stem cells, preferably CD34 positive hematopoietic stem cells.

Particular cell populations, of such as, for example, mononuclear leukocytes, CD3 positive T lymphocytes, CD14 positive monocytes and CD34 positive (hematopoietic) stem cells, can be isolated from a sample obtained from an animal or a human, such as, e.g., a sample of blood or serum, using methods for separating different cell types which are generally known in the art. Examples of such methods are reviewed in Shields et al., 2015. One of the most commonly applied of these separation methods is cell sorting by flow cytometry which utilizes an instrument, such as a fluorescence-activated cell sorter (FACS), wherein cells can be sorted based on physical properties such as size and cell morphology, and also based on detectable labels which can be bound (e.g., via specific antibodies) to particular target structures (antigens) on the cells.

The leukocytes or stem cells are stored at a cell density of 0.001 to 2·10⁸ cells/mL in a storage medium comprising sericin. According to particular embodiments, the cell density is 1 to 5·10⁷ cells/mL, 100 to 1·10⁷ cells/mL, 1·10³ to 5·10⁶, 1·10⁴ to 1·10⁶, for example at 1·10⁵ to 5·10⁵ cells/mL.

### Sericin

The term 'sericin' as used herein refers to sericins which are isolated from natural sources (herein: 'naturally derived sericin'), sericin derivatives, and mixtures thereof. Sericin derivatives can be chemically synthesized or can be obtained by recombinant expression.

Naturally derived sericin that is useful according to the present disclosure can be isolated from the silk fibers produced by the silkworm, *Bombyx mori,* for example from the silk fibers present in silkworm cocoons and raw silk. Naturally derived sericins can include fragments (partial hydrolysates) of the sericin found in silkworm silk fibers which are still functionally active in that they still a capable of reducing storage-associated cell death. Methods which can be used for isolating sericin from *Bombyx mori* silk fibers are described in EP 1 302 544 B1 and in Terada et al., 2002. In preferred embodiments, the sericin is the sericin isolated from *Bombyx mori* silk fibers as described in Terada et al., 2002. In even more preferred embodiments, the sericin is the sericin having Sigma-Aldrich product number S5201 or the sericin of CAS number 474338-68-6.

Chemically synthesized sericins can be obtained using standard methods of protein synthesis known in the art such the t-Boc method or the Fmoc method. In such method, amino acid residues are attached in a stepwise manner starting either from a single amino acid or part of a naturally derived sericin.

Recombinantly expressed sericin can be obtained utilizing standard techniques of recombinant protein expression. For example, a sericin-encoding polynucleotide that expediently is expressible and replicable in a host cell, and typically has the form of a recombinant vector, is introduced into such host cell, the thus modified host cell is cultured and expresses the sericin which then can be isolated from the host cell culture. Particular methods for preparing recombinantly expressed sericin are described in EP 1 302 544 B1.

Sericins which can be used according to the present disclosure include sericins comprising at least one region having the amino acid sequence of SEQ ID NO:1. Preferably the sericins comprise, or consist of, one, two or more repeats of the amino acid sequence of SEQ ID NO:1. Particular examples of such sericins include:
- the sericin 1 precursor of *Bombyx mori* having the amino acid sequence of NCBI reference no. NP_001037506.2 or SEQ ID NO:2,
- the sericin 1-like isoform X1 of *Bombyx mori* having the amino acid sequence of NCBI reference no. XP_012547623.1 or SEQ ID NO:3,
- the sericin 1-like isoform X2 of *Bombyx mori* having the amino acid sequence of NCBI reference no. XP_021205330.1 or SEQ ID NO:4,
- the sericin 1-like isoform X3 of *Bombyx mori* having the amino acid sequence of NCBI reference no. XP_021205333.1 or SEQ ID NO:5,
- the sericin 1-like isoform X5 of *Bombyx mori* having the amino acid sequence of NCBI reference no. XP_021205340.1 or SEQ ID NO:6,
- the sericin 1-like isoform X6 of *Bombyx mori* having the amino acid sequence of NCBI reference no. XP_021205342.1 or SEQ ID NO:7,
- the sericin 1-like isoform X7 of *Bombyx mori* having the amino acid sequence of NCBI reference no. XP_021205348.1 or SEQ ID NO:8,
- the sericin 1A of *Bombyx mori* having the amino acid sequence of EMBL nucleotide sequence database reference no. BAD00698.1 or SEQ ID NO:9,
- the sericin 1A' of *Bombyx mori* having the amino acid sequence of EMBL nucleotide sequence database reference no. BAD00699.1 or SEQ ID NO:10.

Further examples of such sericins are described in EP 1 302 544 B1.

Expediently, the sericin used according to the disclosure has at least 60%, at least 70%, at least 80%, at least 90%, at least 95%, or at least 100% of the activity in reducing storage-associated cell death exhibited by the sericin having Sigma-Aldrich product number S5201, or the sericin of CAS number 474338-68-6, or the sericin isolated from *Bombyx mori* silk fibers as described in Terada et al., 2002. Additionally, said sericin comprises, or consists of, an amino acid sequence having at least 60%, at least 70%, at least 80%, at least 90%, or at least 95% identity with an amino acid sequence selected from SEQ ID NOs:1-10, preferably with the amino acid sequence of SEQ ID NO:1. The degree of amino acid sequence identity can be determined using computer programs for sequence alignment and analysis known in the art such as, e.g., the "BLAST" program accessible from the NCBI webpage (ncbi.nlm.nih.gov/Blast).

Said activity in reducing storage-associated cell death can be determined by a method comprising the following steps:
(1) transferring a cell population selected from human mononuclear leukocytes, human CD3+ T lymphocytes, human CD14+ monocytes and human CD34+ stem cells into a storage medium so as to prepare a suspension of 2x10⁵ cells / mL;
(2) storing the cell suspension in a closed container for 1 week at 4°C;
(3) then mixing the cell suspension with a dead cell stain;
(4) measuring the number of non-stained (i.e. alive) cells in the cell suspension using flow cytometry;
   wherein steps (1)-(4) are performed for each of the following storage media:
   (i) an aqueous solution of 1.432 g/L Na₂HPO₄ · 2 H₂O, 1.255 g/L NaH₂PO₄ · 2 H₂O, 0.194 g/L adenine, 9.405 g/L glucose monohydrate, 0.408 g/L guanosine, 4.210 g/L NaCl and 10.000 g/L mannitol (PAGGS-M),
   (ii) PAGGS-M supplemented with 0.5 weight-% of the tested sericin, relative to the total weight of PAGGS-M plus sericin (PAGGS-M+sericin),
   and wherein the activity in reducing storage-associated cell death corresponds to the difference of the number of non-stained cells after storage in PAGGS-M+sericin minus the number of non-stained cells after storage in PAGGS-M.

In a preferred group of embodiments, the sericin comprises an amino acid sequence having at least 60%, at least 70%, at least 80%, at least 90%, or at least 95% identity with an amino acid sequence selected from SEQ ID NOs:1-10, preferably with the amino acid sequence of SEQ ID NO:1, and has at least 60%, at least 70%, at least 80%, at least 90%, at least 95%, or at least 100% of the activity in reducing storage-associated cell death exhibited by the sericin having Sigma-Aldrich product number S5201, or the sericin of CAS number 474338-68-6, or the sericin isolated from *Bombyx mori* silk fibers as described in Terada et al., 2002, wherein the activity in reducing storage-associated cell death is determined using the above-described methods wherein the cell population used in step (1) are human mononuclear leukocytes.

Expediently, the dead cell stain used in the method for determining activity in reducing storage-associated cell death are suitable for flow cytometry. Such dead cell stains are generally known in the art. Examples for suitable dead cell stains include, but are not limited to, fluorescent DNA intercalating markers such as 7-aminoactinomycin D (7-AAD); Quinolinium, 6-(dimethylamino)-2-[4-[4-(dimethylamino)phenyl]-1,3-butadienyl]-1-ethyl, perchlorate (CAS no. 181885-68-7, available from ThermoFisher Scientific as LDS 751 under #L7595), and the SYTOX^{®} DNA stains available ThermoFisher Scientific as SYTOX^{™} Blue Dead Cell Stain under #S34857, SYTOX^{™} Red Dead Cell Stain under #S34859, SYTOX^{™} Green Dead Cell Stain under #S34860, SYTOX^{™} AADvanced^{™} Dead Cell Stain under #S10274 and S10349, and SYTOX^{™} Orange Dead Cell Stain under #S34861.

Concentrations of sericin in the herein disclosed storage medium which provide the desired degree of reduction in storage-associated cell death at the storage conditions of interest and for the cells of interest can be determined in a routine manner. Specifically, for this purpose one can use a method for determining activity in reducing storage-associated cell death as described hereinabove or in the examples section below and adapting, if necessary, said method so as to use a cell population corresponding to the cells of interest and/or the storage conditions (in particular the storage medium, temperature and duration of storage) of interest.

In particular embodiments, the concentration of sericin in the storage medium is in the range of 0.05-10 weight-%, in particular 0.1-5 weight-%, preferably 0.1-2 weight-%, such as 0.5±0.3 weight-% or 1.0±0.3 weight-%, relative to the total weight of the storage medium.

### Cell storage conditions - media, temperature and duration

Sericin exhibits its activity in reducing storage-associated death of cells in a wide variety of aqueous media, including cell culture (growth) media as well as buffers and other solutions which lack serum and/or one or more of the vitamins, amino acids and/or other compounds (such as insulin, transferrin and selenium) which are usually essential for the culture of animal cells and thus commonly present in cell culture media. This is demonstrated in the examples wherein sericin was shown to reduce storage-associated death of different types of leukocytes and hematopoietic stem cells in the cell culture (growth) medium RPMI 1640, Phosphate-Buffered Saline (PBS) and an aqueous phosphate buffered solution of adenine, glucose, guanosine, NaCl and Mannitol (PAGGS-M).

Accordingly, the storage medium can be a cell culture medium, specifically a growth medium for animal cell culture, for example a commonly used cell culture medium such as RPMI 1640, MEM (Minimal Essential Medium), or DMEM (Dulbecco's modified Eagle's medium), that is supplemented with sericin.

Preferably, however, in the storage medium as provided to the cells, one or more of the vitamins, amino acids and/or other compounds (such as insulin, transferrin and selenium) which are usually essential for the culture of animal cells are absent or present in amounts which are significantly below the amounts typically required for the culture of animal cells (and thus commonly present in cell culture media). Thus, in preferred embodiments, the storage medium as provided to the cells comprises 1/100 or less of the essential amount of valine, or lysine, or leucine, or isoleucine, or threonine, or phenylalanine, or tryptophan, or methionine, or histidine, or glutamine, or cysteine, or arginine, or tyrosine, or folic acid, or niacinamide, or riboflavin, or thiamine, or pyridoxine, or biotin, or vitamin B12, or p-aminobenzoic acid, or D-pantothenic acid, or choline chloride, or myo-inositol, or insulin, or transferrin, or selenium, or one or more than one or all thereof. According to particularly preferred embodiments, the storage medium comprises: 0.0-0.2 mg/L valine, or 0.0-0.4 mg/L lysine, or 0.0-0.5 mg/L leucine, or 0.0-0.5 mg/L isoleucine, or 0.0-0.2 mg/L threonine, or 0.0-0.1 mg/L phenylalanine, or 0.00-0.05 mg/L tryptophan, or 0.0-0.1 mg/L methionine, or 0.0-0.1 mg/L histidine, or 0-3 mg/L glutamine, or 0.0-0.6 mg/L cysteine, or 0-2 mg/L arginine, or 0-1 mg/L tyrosine, or 0.00-0.01 mg/L folic acid, or 0.00-0.01 mg/L niacinamide, or 0.000-0.002 mg/L riboflavin, or 0.00-0.01 mg/L thiamine, or 0.00-0.01 mg/L pyridoxine, or 0-2 µg/L biotin, or 0.00-0.05 µg/L vitamin B12, or 0.00-0.01 mg/L p-aminobenzoic acid, or 0-2 µg/L D-pantothenic acid, or 0.00-0.03 mg/L choline chloride, or 0.00-0.07 mg/L myo-inositol, or 0.0-0.1 mg/L insulin, or 0.00-0.05 mg/L transferrin, or 0.000-0.158 µg/L selenium, or a combination of one or more than one or all thereof.

Unless specified differently, in concentrations of compounds indicated herein as weight / volume, such as concentrations in "mg/L" or "µg/L"", and in concentrations of cells indicated herein as (number of) cells / volume, such as "cells/mL", the volume refers to the volume (specifically the total volume of the medium) at 20°C and 100 kPa.

Preferably, the storage medium comprises no or only minor amounts of blood serum, in particular 0-10 weight-%, such as 0-5 weight-%, preferably 0-1 weight-%, most preferably 0.0-0.2 weight-%, blood serum, relative to the total weight of the medium.

The storage media disclosed herein can be hypertonic, isotonic or hypotonic, wherein isotonic and hypertonic media are preferred and isotonic media are particularly preferred. The storage media can be buffered or unbuffered. The buffered storage media can use buffers which are suitable for cell culture. Such buffers are known in the art and include, but are not limited to, phosphate buffers, buffers based on N,N-bis[2-hydroxyethyl]-2-aminoethanesulfonic acid (BES), 3-(N-morpholino)propanesulfonic acid (MOPS), 2-[[1,3-dihydroxy-2-(hydroxymethyl)propan-2-yl]amino]ethanesulfonic acid (TES), 4-(2-hydroxyethyl)-1-piperazineethanesulfonic acid (HEPES), 3-(N,N-Bis[2-hydroxyethyl]amino)-2-hydroxypropanesulfonic acid, N,N-Bis(2-hydroxyethyl)-3-amino-2-hydroxypropanesulfonic acid (DIPSO), 3-[[1,3-dihydroxy-2-(hydroxymethyl)propan-2-yl]amino]-2-hydroxypropane-1-sulfonic acid (TAPSO), and mixtures thereof.

In preferred embodiments, the medium is phosphate-buffered, in particular with alkali dihydrogenphosphate and dialkali hydrogenphosphate, and most preferably is also isotonic. For example, the phosphate-buffered medium may comprise dialkali hydrogenphosphate and alkali dihydrogenphosphate at a total amount of 10-50 mM, preferably 12-36 mM, and most preferably 24 mM. The alkali of the alkali dihydrogenphosphate and dialkali hydrogenphosphate is sodium, potassium or a mixture thereof, preferably sodium.

In addition to sericin, the medium that is used for storing cells may further comprise adenine, guanosine, glucose, mannitol. For example, the medium may comprise 0.5-4.0 mM adenine, 0.5-4.0 mM guanosine, 20-80 mM glucose, and 20-80 mM mannitol; preferably, 1.0-1.8 mM adenine, 1.0-1.8 mM guanosine, 45-65 mM glucose, and 45-65 mM mannitol; and most preferably 1.4 mM adenine, 1.4 mM guanosine, 47-52 mM glucose, and 55 mM mannitol. Such medium may further comprise NaCl, in particular at a concentration of 40-110mM, preferably 60-85 mM, and most preferably 72 mM.

According to a preferred exemplary embodiment, the storage medium is sericin-containing PAGGS-M.

The cells in storage medium are stored in a non-frozen state, i.e. at a temperature where the storage medium is liquid, and at a temperature of up to 30°C. For example, the cells in storage medium are stored at a temperature in the range of from 0°C to 24°C, such as from 1°C to 20°C, preferably from 1°C to 15°C, more preferably from 1°C to 10°C, even more preferably from 2°C to 8°C, most preferably at 4±2°C. Expediently, the cells are stored in a closed container. Where 'closed container' as used herein refers to a container that is impermeable to microorganisms and viruses, and preferably refers to a container that is airtight.

A storage temperature in the range of from 1°C to 10°C, such as from 2°C to 8°C, specifically at 4±2°C, is particularly preferred when the storage medium is a medium such as, e.g., PAGGS-M that comprises no or only minor amounts of blood serum and wherein vitamins, amino acids and/or other compounds (such as insulin, transferrin and selenium) which are usually essential for the culture of animal cells are absent or present in amounts which are significantly below the amounts typically required for the culture of animal cells, as described herein.

Preferably, there is basically no proliferation of the cells during the storage period. The expression 'basically no proliferation' as used herein means that the cell number at the beginning of the storage period is less than 2-times, preferably less than 1.5-times, most preferably less than 1.2-times, the cell number at the end of the storage period. This can be achieved by the choice of the storage medium and/or the storage temperature. Without wishing to be bound be theory, it is assumed that the rate of cell proliferation will decrease with decreasing storage temperature and/or with the use of storage media wherein one or more of the vitamins, amino acids and other compounds (such as insulin, transferrin and selenium) which are usually essential for the culture of animal cells are absent or present in amounts which are significantly below the amounts typically required for the culture of animal cells. For example, such storage medium may comprise 0.0-0.2 mg/L valine, or 0.0-0.4 mg/L lysine, or 0.0-0.5 mg/L leucine, or 0.0-0.5 mg/L isoleucine, or 0.0-0.2 mg/L threonine, or 0.0-0.1 mg/L phenylalanine, or 0.00-0.05 mg/L tryptophan, or 0.0-0.1 mg/L methionine, or 0.0-0.1 mg/L histidine, or 0-3 mg/L glutamine, or 0.0-0.6 mg/L cysteine, or 0-2 mg/L arginine, or 0-1 mg/L tyrosine, or 0.00-0.01 mg/L folic acid, or 0.00-0.01 mg/L niacinamide, or 0.000-0.002 mg/L riboflavin, or 0.00-0.01 mg/L thiamine, or 0.00-0.01 mg/L pyridoxine, or 0-2 µg/L biotin, or 0.00-0.05 µg/L vitamin B12, or 0.00-0.01 mg/L p-aminobenzoic acid, or 0-2 µg/L D-pantothenic acid, or 0.00-0.03 mg/L choline chloride, or 0.00-0.07 mg/L myo-inositol, or 0.0-0.1 mg/L insulin, or 0.00-0.05 mg/L transferrin, or 0.000-0.158 µg/L selenium, or a combination of one or more than one or all thereof.

The successful choice of a storage medium and/or a storage temperature which results in basically no cell proliferation can be directly and positively verified in a routine manner by determining the cell number at the beginning of the storage period and a the end of the storage period, wherein a cell number at beginning of the storage period that is less than 2-times, preferably less than 1.5-times, most preferably less than 1.2-times, the cell number at the end of the storage period indicates that there is basically no proliferation.

Methods for determining cell numbers, which can be applied for determining the cell number at the beginning of the storage period and cell number at the end of the storage period, are known in the art. Such methods are discussed, e.g. in Vembadi et al., 2019. Examples include the manual counting of cells under the microscope using a hemocytometer such as the Neubauer chamber, electrical impedance-based automated cell counting using a Coulter counter, flow cytometry (wherein cells are detected based on optical parameters such as light scattering and/or fluorescent properties, and digital image analysis using a photomicroscope (wherein cells are detected using a software based on parameters such as size, shape and fluorescent properties). Electrical impedance-based automated cell counting using a Coulter counter and flow cytometry are methods which are particularly preferred for high-throughput applications.

The use of sericin and the storage medium disclosed herein can be applied for storing leukocytes or stem cells at a temperature of up to 30°C where the storage medium is liquid for at least 24 hours, at least 2 days, at least 4 days, at least 1 week, at least 2 weeks, at least 3 weeks, or up to 2 weeks, up to 3 weeks, up to 4 weeks, up to 5 weeks, or up to 6 weeks; for example for a period of at least 24 hours up to 6 weeks, or for a period of at least 2 days up to 5 weeks, or for a period of at least 4 days of up to 4 weeks.

In the herein disclosed method for storing cells, the cells suspended in storage medium are stored, at a temperature of up to 30°C where the storage medium is liquid, for a period of at least 24 hours up to 6 weeks, for example for a period of at least 24 hours up to 6 weeks, or for a period of at least 2 days up to 5 weeks, or for a period of at least 4 days of up to 4 weeks.

The present disclosure further relates to a dry medium or a medium concentration. Said dry medium or medium concentrate can be reconstituted with a solvent so as to form a storage medium as disclosed herein. The solvent is preferably an aqueous solvent, most preferably water.

Expediently, the herein disclosed storage medium, dry medium and medium concentrate as well as the solvent used for reconstituting the dry medium or medium concentrate are sterile so as to prevent contamination.

As a further measure to prevent contamination, at least one agent selected from antibiotics and antifugal agents can be added. Suitable antibiotics and antifugal agents include, but are not limited to, amphotericin B, gentamycin (e.g. as gentamycin sulfate), penicillin, streptomycin, and kanamycin (e.g., as kanamycin sulfate).

The disclosure further comprises the following embodiments E1-E54:
E1: Use of sericin as a medium additive for reducing storage-associated death of cells stored in an aqueous storage medium at a cell density of 0.001 to 2·10⁸ cells/mL and at a temperature of up to 30°C where the storage medium is liquid, wherein the cells are selected from leukocytes and stem cells, preferably of human origin.
E2: Method for storing cells selected from leukocytes and stem cells, preferably of human origin, the method comprising:
   (i) preparing a suspension of the cells with a cell density of 0.001 to 2·10⁸ cells/mL in an aqueous storage medium comprising sericin;
   (ii) storing the cell suspension at a temperature of up to 30°C where the storage medium is liquid for a period of at least 24 hours up to 6 weeks.
E3: The use of E1, or the method of E2, wherein the concentration of the sericin in the storage medium is in the range of 0.05-10 weight-%, in particular 0.1-5 weight-%, preferably 0.1-2 weight-%, such as 0.5±0.3 weight-% or 1.0±0.3 weight-%, relative to the total weight of the storage medium.
E4: The use of E1 or E3, or the method of E2 or E3, wherein the storage medium and/or the storage temperature are chosen such that there is basically no proliferation of the cells during the storage period, as determined in that the cell number at the beginning of the storage period is less than 2-times, preferably less than 1.5-times, most preferably less than 1.2-times, the cell number at the end of the storage period.
E5: The use of any one of E1, E3 and E4, or the method of any one of E2-E4, wherein the storage medium comprises:
   (i) 0.0-0.2 mg/L valine, or
   (ii) 0.0-0.4 mg/L lysine, or
   (iii) 0.0-0.5 mg/L leucine, or
   (iv) 0.0-0.5 mg/L isoleucine, or
   (v) 0.0-0.2 mg/L threonine, or
   (vi) 0.0-0.1 mg/L phenylalanine, or
   (vii) 0.00-0.05 mg/L tryptophan, or
   (viii) 0.0-0.1 mg/L methionine, or
   (ix) 0.0-0.1 mg/L histidine, or
   (x) 0-3 mg/L glutamine, or
   (xi) 0.0-0.6 mg/L cysteine, or
   (xii) 0-2 mg/L arginine, or
   (xiii) 0-1 mg/L tyrosine, or
   (xiv) 0.00-0.01 mg/L folic acid, or
   (xv) 0.00-0.01 mg/L niacinamide, or
   (xvi) 0.000-0.002 mg/L riboflavin, or
   (xvii) 0.00-0.01 mg/L thiamine, or
   (xviii) 0.00-0.01 mg/L pyridoxine, or
   (xix) 0-2 µg/L biotin, or
   (xx) 0.00-0.05 µg/L vitamin B12, or
   (xxi) 0.00-0.01 mg/L p-aminobenzoic acid, or
   (xxii) 0-2 µg/L D-pantothenic acid, or
   (xxiii) 0.00-0.03 mg/L choline chloride, or
   (xxiv) 0.00-0.07 mg/L myo-inositol, or
   (xxv) 0.0-0.1 mg/L insulin, or
   (xxvi) 0.00-0.05 mg/L transferrin, or
   (xxvii) 0.000-0.158 µg/L selenium, or
   (xxviii) one or more than one or all of (i)-(xxvii).
E6: The use of any one of E1 and E3-E5, or the method of any one of E2-E5, wherein the storage medium comprises 0-10 weight-%, such as 0-5 weight-%, preferably 0-1 weight-%, most preferably 0.0-0.2 weight-%, blood serum, relative to the total weight of the storage medium.
E7: The use of any one of E1 and E3-E6, or the method of any one of E2-E6, wherein the storage medium is hypertonic, isotonic, or hypotonic, preferably isotonic or hypertonic, more preferably isotonic, and is buffered or unbuffered, preferably phosphate-buffered, and most preferably is both isotonic and phosphate-buffered.
E8: The use or the method of E7, wherein the storage medium is buffered with alkali dihydrogenphosphate and dialkali hydrogenphosphate, wherein the alkali is sodium, potassium or a mixture thereof, preferably sodium.
E9: The use of any one of E1 and E3-E8, or the method of any one of E2-E8, wherein the storage medium comprises adenine, guanosine, glucose, and mannitol.
E10: The use or the method of any one of E7-E9, wherein the storage medium comprises:
   - 0.5-4.0 mM adenine,
   - 0.5-4.0 mM guanosine,
   - 20-80 mM glucose, and
   - 20-80 mM mannitol,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E11: The use or the method of any one of E7-E10, wherein the storage medium comprises:
   - dialkali hydrogen phosphate and alkali dihydrogenphosphate at a total amount of 10-50 mM, wherein the alkali is sodium, potassium or a mixture thereof, preferably sodium,
   - 0.5-4.0 mM adenine,
   - 0.5-4.0 mM guanosine,
   - 20-80 mM glucose,
   - 20-80 mM mannitol, and
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E12: The use or the method of any one of E7-E11, wherein the storage medium comprises:
   - dialkali hydrogenphosphate and alkali dihydrogenphosphate at a total amount of 10-50 mM, wherein the alkali is sodium, potassium or a mixture thereof, preferably sodium,
   - 0.5-4.0 mM adenine,
   - 0.5-4.0 mM guanosine,
   - 20-80 mM glucose,
   - 20-80 mM mannitol, and
   - 0.05-10 weight-% of the sericin, relative to the total weight of the storage medium,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E13: The use or the method of any one of E7-E12, wherein the storage medium comprises:
   - 1.0-1.8 mM adenine,
   - 1.0-1.8 mM guanosine,
   - 45-65 mM glucose, and
   - 45-65 mM mannitol,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E14: The use or the method of any one of E7-E13, wherein the storage medium comprises:
   - dialkali hydrogen phosphate and alkali dihydrogenphosphate at a total amount of 12-36 mM, wherein the alkali is sodium, potassium or a mixture thereof, preferably sodium,
   - 1.0-1.8 mM adenine,
   - 1.0-1.8 mM guanosine,
   - 45-65 mM glucose, and
   - 45-65 mM mannitol,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E15: The use or the method of any one of E7-E14, wherein the storage medium comprises:
   - dialkali hydrogen phosphate and alkali dihydrogenphosphate at a total amount of 12-36 mM, wherein the alkali is sodium, potassium or a mixture thereof, preferably sodium,
   - 1.0-1.8 mM adenine,
   - 1.0-1.8 mM guanosine,
   - 45-65 mM glucose,
   - 45-65 mM mannitol, and
   - 0.1-5 weight-% of the sericin, relative to the total weight of the storage medium,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E16: The use or the method of any one of E7-E15, wherein the storage medium comprises:
   - 1.4 mM adenine,
   - 1.4 mM guanosine,
   - 47-52 mM glucose, and
   - 55 mM mannitol,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E17: The use or the method of any one of E7-E16, wherein the storage medium comprises:
   - dialkali hydrogenphosphate and alkali dihydrogenphosphate at a total amount of 24 mM, wherein the alkali is sodium, potassium or a mixture thereof, preferably sodium,
   - 1.4 mM adenine,
   - 1.4 mM guanosine,
   - 47-52 mM glucose, and
   - 55 mM mannitol,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E18: The use or the method of any one of E7-E17, wherein the storage medium comprises:
   - dialkali hydrogen phosphate and alkali dihydrogenphosphate at a total amount of 24 mM, wherein the alkali is sodium, potassium or a mixture thereof, preferably sodium,
   - 1.4 mM adenine,
   - 1.4 mM guanosine,
   - 47-52 mM glucose,
   - 55 mM mannitol, and
   - 0.5±0.3 weight-% or 1.0±0.3 weight-% of the sericin, relative to the total weight of the storage medium,
      and preferably comprises:
      (i) 0.0-0.2 mg/L valine, or
      (ii) 0.0-0.4 mg/L lysine, or
      (iii) 0.0-0.5 mg/L leucine, or
      (iv) 0.0-0.5 mg/L isoleucine, or
      (v) 0.0-0.2 mg/L threonine, or
      (vi) 0.0-0.1 mg/L phenylalanine, or
      (vii) 0.00-0.05 mg/L tryptophan, or
      (viii) 0.0-0.1 mg/L methionine, or
      (ix) 0.0-0.1 mg/L histidine, or
      (x) 0-3 mg/L glutamine, or
      (xi) 0.0-0.6 mg/L cysteine, or
      (xii) 0-2 mg/L arginine, or
      (xiii) 0-1 mg/L tyrosine, or
      (xiv) 0.00-0.01 mg/L folic acid, or
      (xv) 0.00-0.01 mg/L niacinamide, or
      (xvi) 0.000-0.002 mg/L riboflavin, or
      (xvii) 0.00-0.01 mg/L thiamine, or
      (xviii) 0.00-0.01 mg/L pyridoxine, or
      (xix) 0-2 µg/L biotin, or
      (xx) 0.00-0.05 µg/L vitamin B12, or
      (xxi) 0.00-0.01 mg/L p-aminobenzoic acid, or
      (xxii) 0-2 µg/L D-pantothenic acid, or
      (xxiii) 0.00-0.03 mg/L choline chloride, or
      (xxiv) 0.00-0.07 mg/L myo-inositol, or
      (xxv) 0.0-0.1 mg/L insulin, or
      (xxvi) 0.00-0.05 mg/L transferrin, or
      (xxvii) 0.000-0.158 µg/L selenium, or
      (xxviii) one or more than one or all of (i)-(xxvii), and
      further preferably comprises 0-10 weight-%, such as 0-5 weight-%, particularly 0-1 weight-%, especially 0.0-0.2 weight-%, blood serum, relative to the total weight of the storage medium.
E19: The use or the method of any one of E7-E18, wherein the storage medium further comprises sodium chloride, preferably at an concentration of 40-110mM, more preferably 60-85 mM, and most preferably 73 mM.
E20: The use of any one of E1 and E3-E19, or the method of any one of E2-E19, wherein the cells in storage medium are stored at a temperature in the range of from 0°C to 24°C, such as from 1°C to 20°C, preferably from 1°C to 15°C, more preferably from 1°C to 10°C, even more preferably from 2°C to 8°C, most preferably at 4±2°C.
E21: The use of any one of E1 and E3-E20, or the method of any one of E2-E20, wherein the sericin comprises at least one region having the amino acid sequence of SEQ ID NO:1.
E22: The use of any one of E1 and E3-E22, or the method of any one of E2-E22, wherein the sericin:
   (i) is selected from naturally derived sericins and sericin derivatives as well as mixtures thereof,
   (ii) comprises an amino acid sequence having at least 60% identity with an amino acid sequence selected from SEQ ID NOs:1-10, preferably with the amino acid sequence of SEQ ID NO:1, and
   (iii) has at least 60% of the activity in reducing storage-associated cell death exhibited by the sericin having Sigma-Aldrich product number S5201 or the sericin of CAS number 474338-68-6 or the sericin isolated from *Bombyx mori* silk fibers as described in Terada et al., Cytotechnology 2002, 40:3-12.
E23: The use or the method of E22, wherein the activity in reducing storage-associated cell death is determined by the method comprising the following steps:
   (1) transferring a cell population selected from human mononuclear leukocytes, human CD3+ T lymphocytes, human CD14+ monocytes and human CD34+ stem cells into a storage medium so as to prepare a suspension of 2x10⁵ cells / mL;
   (2) storing the cell suspension in a closed container for 1 week at 4°C;
   (3) then mixing the cell suspension with a dead cell stain;
   (4) measuring the number of non-stained cells in the cell suspension using flow cytometry;
      wherein steps (1)-(4) are performed for each of the following storage media:
      (i) 1.432 g/L Na₂HPO₄ · 2 H₂O, 1.255 g/L NaH₂PO₄ · 2 H₂O, 0.194 g/L adenine, 9.405 g/L glucose monohydrate, 0.408 g/L guanosine, 4.210 g/L NaCl, 10.000 g/L mannitol, in water (PAGGS-M),
      (ii) PAGGS-M supplemented with 0.5 weight-% of the tested sericin, relative to the total weight of PAGGS-M plus sericin (PAGGS-M+sericin);
      and wherein the activity in reducing storage-associated cell death corresponds to the difference of the number of non-stained cells after storage in PAGGS-M+sericin minus the number of non-stained cells after storage in PAGGS-M.
E24: The use of any one of E1 and E3-E23, or the method of any one of E2-E23, wherein the sericin comprises an amino acid sequence selected from SEQ ID NOs:2-10.
E25: The use of any one of E1 and E3-E24, or the method of any one of E2-E24, wherein the sericin is the sericin having Sigma-Aldrich product number S5201.
E26: The use of any one of E1 and E3-E24, or the method of any one of E2-E24, wherein the sericin is the sericin of CAS number 474338-68-6.
E27: The use of any one of E1 and E3-E24, or the method of any one of E2-E24, wherein the sericin is the sericin isolated from *Bombyx mori* silk fibers as described in Terada et al., Cytotechnology 2002, 40:3-12.
E28: The use of any one of E1 and E3-E27, or the method of any one of E2-E27, wherein the leukocytes are selected from T lymphocytes, B lymphocytes, NK cells, monocytes, dendritic cells and granulocytes, and preferably are T lymphocytes or monocytes, most preferably CD3 positive T lymphocytes or CD14 positive monocytes;
   wherein the stem cells are hematopoietic stem cells, preferably CD34 positive hematopoietic stem cells.
E29: A medium for storing cells selected from leukocytes and stem cells, wherein the medium is an aqueous solution comprising sericin and:
   (i) 0.0-0.2 mg/L valine, or
   (ii) 0.0-0.4 mg/L lysine, or
   (iii) 0.0-0.5 mg/L leucine, or
   (iv) 0.0-0.5 mg/L isoleucine, or
   (v) 0.0-0.2 mg/L threonine, or
   (vi) 0.0-0.1 mg/L phenylalanine, or
   (vii) 0.00-0.05 mg/L tryptophan, or
   (viii) 0.0-0.1 mg/L methionine, or
   (ix) 0.0-0.1 mg/L histidine, or
   (x) 0-3 mg/L glutamine, or
   (xi) 0.0-0.6 mg/L cysteine, or
   (xii) 0-2 mg/L arginine, or
   (xiii) 0-1 mg/L tyrosine, or
   (xiv) 0.00-0.01 mg/L folic acid, or
   (xv) 0.00-0.01 mg/L niacinamide, or
   (xvi) 0.000-0.002 mg/L riboflavin, or
   (xvii) 0.00-0.01 mg/L thiamine, or
   (xviii) 0.00-0.01 mg/L pyridoxine, or
   (xix) 0-2 µg/L biotin, or
   (xx) 0.00-0.05 µg/L vitamin B12, or
   (xxi) 0.00-0.01 mg/L p-aminobenzoic acid, or
   (xxii) 0-2 µg/L D-pantothenic acid, or
   (xxiii) 0.00-0.03 mg/L choline chloride, or
   (xxiv) 0.00-0.07 mg/L myo-inositol, or
   (xxv) 0.0-0.1 mg/L insulin, or
   (xxvi) 0.00-0.05 mg/L transferrin, or
   (xxvii) 0.000-0.158 µg/L selenium, or
   (xxviii) one or more than one or all of (i)-(xxvii).
E30: The medium of E29, wherein the medium comprises 0-10 weight-%, such as 0-5 weight-%, preferably 0-1 weight-%, most preferably 0.0-0.2 weight-%, blood serum, relative to the total weight of the medium.
E31: The medium of E29 or E30, wherein the concentration of the sericin in the medium is in the range of 0.05-10 weight-%, in particular 0.1-5 weight-%, preferably 0.1-2 weight-%, such as 0.5±0.3 weight-% or 1.0±0.3 weight-%, relative to the total weight of the medium.
E32: The medium of any one of E29-E31, wherein the medium is a hypertonic, isotonic, or hypotonic, buffered or unbuffered solution, preferably an isotonic or hypertonic, more preferably isotonic, solution, and most preferably an isotonic phosphate-buffered solution.
E33: The medium of E32, wherein the medium is buffered with alkali dihydrogenphosphate and dialkali hydrogenphosphate, wherein the alkali is sodium, potassium or a mixture thereof, preferably sodium.
E34: The medium of any one of E29-E33, wherein the medium comprises adenine, guanosine, glucose, mannitol.
E35: The medium of any one of E29-E34, wherein the medium comprises:
   - 0.5-4.0 mM adenine,
   - 0.5-4.0 mM guanosine,
   - 20-80 mM glucose,
   - 20-80 mM mannitol, and
   - preferably 0-10 weight-%, such as 0-5 weight-%, particularly 0-1 weight-%, especially 0.0-0.2 weight-%, blood serum, relative to the total weight of the medium.
E36: The medium of any one of E29-E35, wherein the medium comprises:
   - dialkali hydrogen phosphate and alkali dihydrogenphosphate at a total amount of 10-50 mM, wherein the alkali is sodium, potassium or a mixture thereof, preferably sodium,
   - 0.5-4.0 mM adenine,
   - 0.5-4.0 mM guanosine,
   - 20-80 mM glucose,
   - 20-80 mM mannitol, and
   - preferably 0-10 weight-%, such as 0-5 weight-%, particularly 0-1 weight-%, especially 0.0-0.2 weight-%, blood serum, relative to the total weight of the medium.
E37: The medium of any one of E29-E36, wherein the medium comprises:
   - dialkali hydrogen phosphate and alkali dihydrogenphosphate at a total amount of 10-50 mM, wherein the alkali is sodium, potassium or a mixture thereof, preferably sodium,
   - 0.5-4.0 mM adenine,
   - 0.5-4.0 mM guanosine,
   - 20-80 mM glucose,
   - 20-80 mM mannitol,
   - 0.05-10 weight-% of the sericin, relative to the total weight of the medium, and
   - preferably 0-10 weight-%, such as 0-5 weight-%, particularly 0-1 weight-%, especially 0.0-0.2 weight-%, blood serum, relative to the total weight of the medium.
E38: The medium of any one of E29-E37, wherein the medium comprises:
   - 1.0-1.8 mM adenine,
   - 1.0-1.8 mM guanosine,
   - 45-65 mM glucose,
   - 45-65 mM mannitol, and
   - preferably 0-10 weight-%, such as 0-5 weight-%, particularly 0-1 weight-%, especially 0.0-0.2 weight-%, blood serum, relative to the total weight of the medium.
E39: The medium of any one of E29-E38, wherein the medium comprises:
   - dialkali hydrogen phosphate and alkali dihydrogenphosphate at a total amount of 12-36 mM, wherein the alkali is sodium, potassium or a mixture thereof, preferably sodium,
   - 1.0-1.8 mM adenine,
   - 1.0-1.8 mM guanosine,
   - 45-65 mM glucose,
   - 45-65 mM mannitol, and
   - preferably 0-10 weight-%, such as 0-5 weight-%, particularly 0-1 weight-%, especially 0.0-0.2 weight-%, blood serum, relative to the total weight of the medium.
E40: The medium of any one of E29-E39, wherein medium comprises:
   - dialkali hydrogen phosphate and alkali dihydrogenphosphate at a total amount of 12-36 mM, wherein the alkali is sodium, potassium or a mixture thereof, preferably sodium,
   - 1.0-1.8 mM adenine,
   - 1.0-1.8 mM guanosine,
   - 45-65 mM glucose,
   - 45-65 mM mannitol,
   - 0.1-5 weight-% of the sericin, relative to the total weight of the medium, and
   - preferably 0-10 weight-%, such as 0-5 weight-%, particularly 0-1 weight-%, especially 0.0-0.2 weight-%, blood serum, relative to the total weight of the medium.
E41: The medium of any one of E29-E40, wherein the medium comprises:
   - 1.4 mM adenine,
   - 1.4 mM guanosine,
   - 47-52 mM glucose,
   - 55 mM mannitol, and
   - preferably 0-10 weight-%, such as 0-5 weight-%, particularly 0-1 weight-%, especially 0.0-0.2 weight-%, blood serum, relative to the total weight of the medium.
E42: The medium of any one of E29-E41, wherein the medium comprises:
   - dialkali hydrogenphosphate and alkali dihydrogenphosphate at a total amount of 24 mM, wherein the alkali is sodium, potassium or a mixture thereof, preferably sodium,
   - 1.4 mM adenine,
   - 1.4 mM guanosine,
   - 47-52 mM glucose,
   - 55 mM mannitol, and
   - preferably 0-10 weight-%, such as 0-5 weight-%, particularly 0-1 weight-%, especially 0.0-0.2 weight-%, blood serum, relative to the total weight of the medium.
E43: The medium of any one of E29-E42, wherein the medium comprises:
   - dialkali hydrogenphosphate and alkali dihydrogenphosphate at a total amount of 24 mM, wherein the alkali is sodium, potassium or a mixture thereof, preferably sodium,
   - 1.4 mM adenine,
   - 1.4 mM guanosine,
   - 47-52 mM glucose,
   - 55 mM mannitol,
   - 0.5±0.3 weight-% or 1.0±0.3 weight-% of the sericin, relative to the total weight of the medium, and
   - preferably 0-10 weight-%, such as 0-5 weight-%, particularly 0-1 weight-%, especially 0.0-0.2 weight-%, blood serum, relative to the total weight of the medium.
E44: The medium of any one of E29-E43, wherein the medium further comprises sodium chloride, preferably at an concentration of 40-110mM, more preferably 60-85 mM, and most preferably 73 mM.
E45: The medium of any one of E29-E44, wherein the sericin comprises at least one region having the amino acid sequence of SEQ ID NO:1.
E46: The medium of any one of E29-E45, wherein the sericin:
   (i) is selected from naturally derived sericins and sericin derivatives as well as mixtures thereof,
   (ii) comprises an amino acid sequence having at least 60% identity with an amino acid sequence selected from SEQ ID NOs:1-10, preferably with the amino acid sequence of SEQ ID NO:1, and
   (iii) has at least 60% of the activity in reducing storage-associated cell death exhibited by the sericin having Sigma-Aldrich product number S5201 or the sericin of CAS number 474338-68-6 or the sericin isolated from *Bombyx mori* silk fibers as described in Terada et al., Cytotechnology 2002, 40:3-12.
E47: The medium of E46, wherein the activity in reducing storage-associated cell death is determined by the method comprising the following steps:
   (1) transferring a cell population selected from human mononuclear leukocytes, human CD3+ T lymphocytes, human CD14+ monocytes and human CD34+ stem cells into a storage medium so as to prepare a suspension of 2x10⁵ cells / mL;
   (2) storing the cell suspension in a closed container for 1 week at 4°C;
   (3) then mixing the cell suspension with a dead cell stain;
   (4) measuring the number of non-stained cells in the cell suspension using flow cytometry;
      wherein steps (1)-(4) are performed for each of the following storage media:
      (i) an aqueous solution of 1.432 g/L Na₂HPO₄ · 2 H₂O, 1.255 g/L NaH₂PO₄ · 2 H₂O, 0.194 g/L adenine, 9.405 g/L glucose monohydrate, 0.408 g/L guanosine, 4.210 g/L NaCl and 10.000 g/L mannitol (PAGGS-M),
      (ii) PAGGS-M supplemented with 0.5 weight-% of the tested sericin, relative to the total weight of PAGGS-M plus sericin (PAGGS-M+sericin);
      and wherein the activity in reducing storage-associated cell death corresponds to the difference of the number of non-stained cells after storage in PAGGS-M+sericin minus the number of non-stained cells after storage in PAGGS-M.
E48: The medium of any one of E29-E47, wherein the sericin comprises an amino acid sequence selected from SEQ ID NOs:2-10.
E49: The medium of any one of E29-E48, wherein the sericin is the sericin having Sigma-Aldrich product number S5201.
E50: The medium of any one of E29-E48, wherein the sericin is the sericin of CAS number 474338-68-6.
E51: The medium of any one of E29-E48, wherein the sericin is the sericin isolated from *Bombyx mori* silk fibers as described in Terada et al., Cytotechnology 2002, 40:3-12.
E52: A dry medium formulation or a medium concentrate that can be reconstituted with a solvent, preferably water, so as to form a medium as defined in any one of E1-E51.
E53: A cell suspension in a medium as defined in any one of E1-E51 having a cell density of 0.001 to 2·10⁸ cells/mL, wherein the cells are selected from leukocytes and stem cells, preferably of human origin.
E54: The cell suspension of E53, wherein the leukocytes are selected from T lymphocytes, B lymphocytes, NK cells, monocytes, dendritic cells and granulocytes, and preferably are T lymphocytes or monocytes, most preferably CD3 positive T lymphocytes or CD14 positive monocytes;
   wherein the stem cells are hematopoietic stem cells,
   preferably CD34 positive hematopoietic stem cells.

### DESCRIPTION OF FIGURES

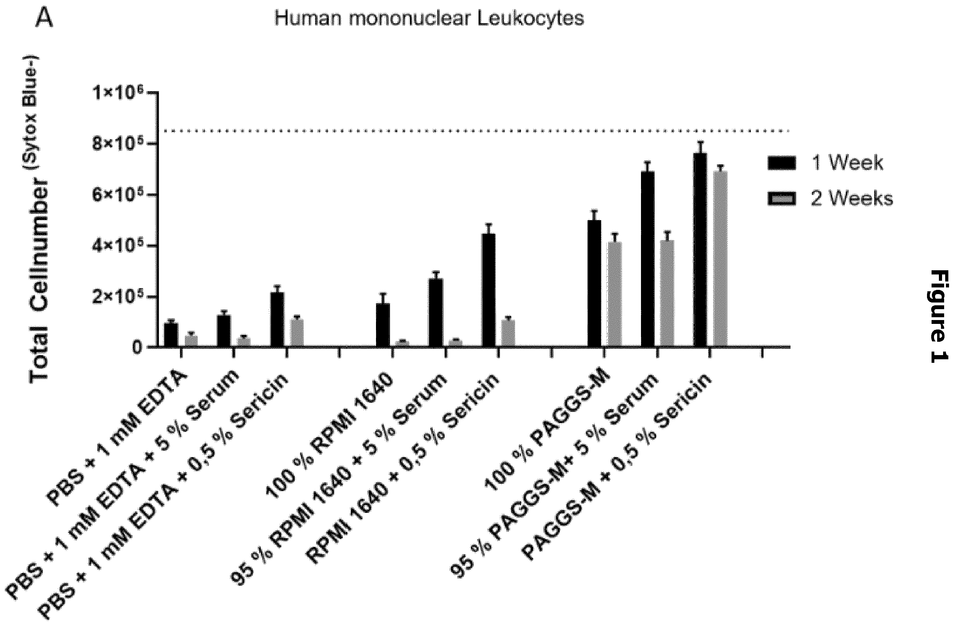

**Figures 1-4** show total numbers of viable cells as measured by flow cytometry based on a dead cell stain. Dotted line indicates the initial number of viable cells, i.e. the viable cell number prior to storage.
**Figure 1** shows mean and standard error of mean (of three experiments with triplicate wells) of the number of viable human mononuclear leukocytes. Black bars indicate viable cell numbers after 1 week of storage at 4°C in the respective storage medium (which is indicated at the x-axis), and grey bars indicate viable cell numbers after 2 weeks of storage at 4°C in the respective storage medium.

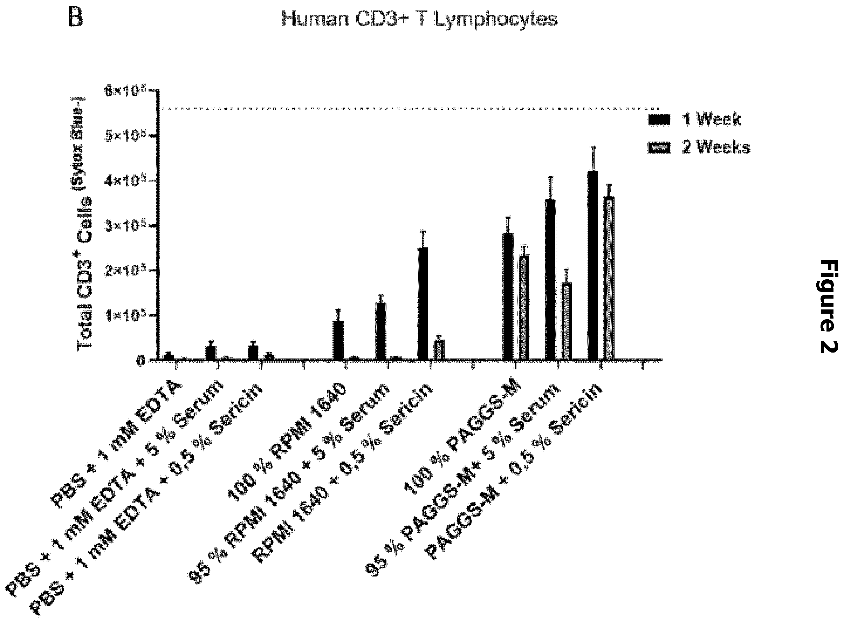

**Figure 2** shows mean and standard error of mean (of three experiments with triplicate wells) of the number of viable human CD3+ leucocytes. Black bars indicate viable cell numbers after 1 week of storage at 4°C in the respective storage medium (which is indicated at the x-axis), and grey bars indicate viable cell numbers after 2 weeks of storage at 4°C in the respective storage medium.

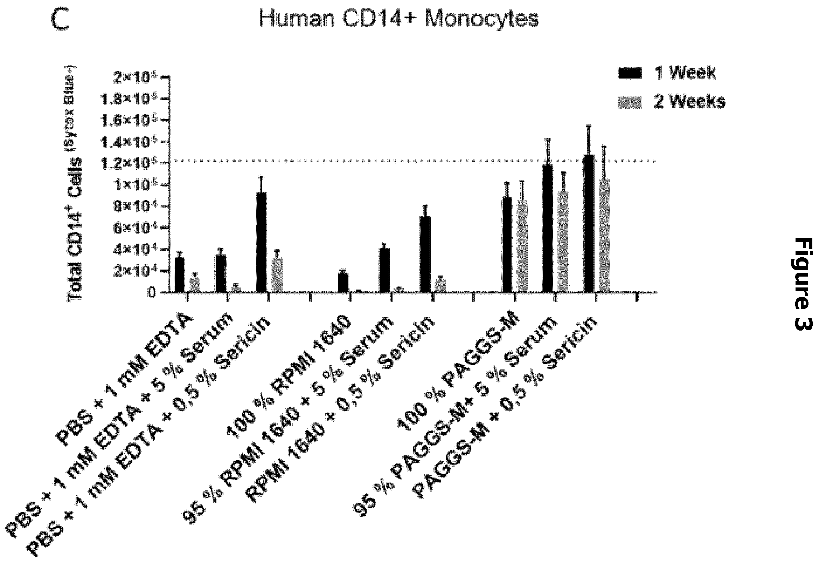

**Figure 3** shows mean and standard error of mean (of three experiments with triplicate wells) of the number of viable human CD14+ monocytes. Black bars indicate viable cell numbers after 1 week of storage at 4°C in the respective storage medium (which is indicated at the x-axis), and grey bars indicate viable cell numbers after 2 weeks of storage at 4°C in the respective storage medium.

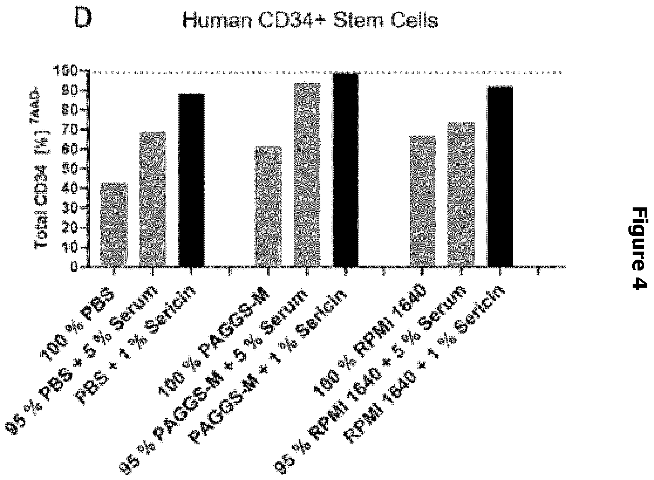

**Figure 4** shows the number of viable human CD34+ stem cells of a single experiment. Bars indicate viable cell numbers after 1 week of storage at 4°C in the respective storage medium (which is indicated at the x-axis).

### EXAMPLES

The four cell populations mononuclear leukocytes, CD3+ T lymphocytes, CD14+ monocytes and CD34+ stem cells were specifically analyzed in PBMC or leukocytapheresis samples by flow cytometry after surface staining of the respective surface markers with fluorochrome-labelled monoclonal antibodies.

Samples were immediately transferred into a storage medium so as to prepare a suspension of 2x10⁵ cells / mL. 1 ml of each cell suspension was stored in a closed 1.5 mL centrifuge vial for 1 week or for 2 weeks at 4°C in a fridge. The total number of viable cells was determined prior to and after this storage period as follows:
200 µL of each cell suspension was transferred into a 96-well V-bottom microtiter plate, mixed with 1 µL of a dead cell stain (Sytox blue vital dye, Life Technologies, cat. no. S34857) and stored for at room temperature for 5 min. The number of viable cells was determined by counting Sytox blue stained (i.e. dead) and non-stained (i.e. alive) cells using flow cytometry so as to determine their absolute and relative numbers.

The following storage media were tested for each of the four cell populations:
(1) PBS (137 mM NaCl, 2.7 mM KCI, 10 mM Na₂HPO₄, 1.8 mM KH₂PO₄, pH 7.4, in water), 1 mM EDTA
(2) PBS, 1 mM EDTA, 5 weight-% human serum (Bio&Sell, cat. no. HUAB.SE.0100)
(3) PBS, 1mM EDTA, 0.5 weight-% sericin
(4) RPMI 1640 (0.1 g/L Calcium Nitrate • 4H₂O, 0.04884 g/L Magnesium Sulfate (anhydrous), 0.4 g/L Potassium Chloride, 2 g/L Sodium Bicarbonate, 6 g/L Sodium Chloride, 0.8 g/L Sodium Phosphate Dibasic (anhydrous), 0.2 g/L L-Arginine, 0.05 g/L L-Asparagine (anhydrous), 0.02 g/L L-Aspartic Acid, 0.0652 g/L L-Cystine • 2HCl, 0.02 g/L L-Glutamic Acid, 0.3 g/L L-Glutamine, 0.01 g/L Glycine, 0.015 g/L L-Histidine, 0.02 g/L Hydroxy-L-Proline, 0.05 g/L L-Isoleucine, 0.05 g/L L-Leucine, 0.04 g/L L-Lysine • HCl, 0.015 g/L L-Methionine, 0.015 g/L L-Phenylalanine, 0.02 g/L L-Proline, 0.03 g/L L-Serine, 0.02 g/L L-Threonine, 0.005 g/L L-Tryptophan, 0.02883 g/L L-Tyrosine • 2Na • 2H₂O, 0.02 g/L L-Valine, 0.0002 g/L D-Biotin, 0.003 g/L Choline Chloride, 0.001 g/L Folic Acid, 0.035 g/L *myo*-Inositol, 0.001 g/L Niacinamide, 0.001 g/L *p*-Aminobenzoic Acid, 0.00025 g/L D-Pantothenic Acid (hemicalcium), 0.001 g/L Pyridoxine • HCl, 0.0002 g/L Riboflavin, 0.001 g/L Thiamine • HCl, 0.000005 g/L Vitamin B₁₂, 2 g/L D-Glucose, 0.001 g/L Glutathione (reduced), 0.0053 g/L Phenol Red • Na)
(5) RPMI 1640, 5 weight-% serum
(6) RPMI 1640, 0.5 weight-% sericin
(7) PAGGS-M (1.432 g/L Na₂HPO₄ · 2 H₂O, 1.255 g/L NaH₂PO₄ · 2 H₂O, 0.194 g/L adenine, 9.405 g/L glucose monohydrate, 0.408 g/L guanosine, 4.210 g/L NaCl, 10.000 g/L mannitol, in water)
(8) PAGGS-M, 5 weight-% serum
(9) PAGGS-M, 0.5 weight-% sericin
   (The sericin used in these storage media was purchased from of Sigma-Aldrich, St. Louis, Missouri, U.S.A. (#S5201). This sericin is isolated from *Bombyx mori* cocoons according to Terada et al., 2002 and has CAS no. 474338-68-6.)

The results are shown in the form of bar charts in Figures 1-4. Surprisingly, the presence of only 0.5 weight-% sericin significantly reduced storage-associated cell death in the very different tested media and in all of the different tested cell types. Notably, the tested cell types includes cells having different nutrient requirements namely non-proliferating CD14+ monocytes, CD3+ T lymphocytes which are capable of proliferation, and CD34+ stem cells which can expand and differentiate into a variety different blood cell types). In nearly all experiments, the sericin-containing storage media preserved cell viability significantly better than the serum-containing storage media. Particularly good results were observed with sericin-containing PAGGS-M where no or only a rather small drop in viable cell numbers was observed, even after two weeks of storage.

### LIST OF REFERENCES

EP1302544B1
EP1335018A1
Dong et al., 2019, Int J Biol Macromol, 132, 1121-1130
Pasovic et al., 2018, Scientific Reports, 8, 5688
Terada et al., 2002, Cytotechnology, 40, 3-12
Shields et al., 2015, Lab Chip, 15(5), 1230-1249
Vembadi et al., 2019, Front Bioeng Biotechnol, 7, 147

## Claims

1. Use of sericin as a medium additive for reducing storage-associated death of cells stored in an aqueous storage medium at a cell density of 0.001 to 2·10⁸ cells/mL and at a temperature of up to 30°C where the storage medium is liquid, wherein the cells are selected from leukocytes and stem cells, preferably of human origin.

2. Method for storing cells selected from leukocytes and stem cells, preferably of human origin, the method comprising:
(i) preparing a suspension of the cells with a cell density of 0.001 to 2·10⁸ cells/mL in an aqueous storage medium comprising sericin;
(ii) storing the cell suspension at a temperature of up to 30°C where the storage medium is liquid for a period of at least 24 hours up to 6 weeks.

3. The use of claim 1, or the method of claim 2, wherein the storage medium comprises:
(i) 0.0-0.2 mg/L valine, or
(ii) 0.0-0.4 mg/L lysine, or
(iii) 0.0-0.5 mg/L leucine, or
(iv) 0.0-0.5 mg/L isoleucine, or
(v) 0.0-0.2 mg/L threonine, or
(vi) 0.0-0.1 mg/L phenylalanine, or
(vii) 0.00-0.05 mg/L tryptophan, or
(viii) 0.0-0.1 mg/L methionine, or
(ix) 0.0-0.1 mg/L histidine, or
(x) 0-3 mg/L glutamine, or
(xi) 0.0-0.6 mg/L cysteine, or
(xii) 0-2 mg/L arginine, or
(xiii) 0-1 mg/L tyrosine, or
(xiv) 0.00-0.01 mg/L folic acid, or
(xv) 0.00-0.01 mg/L niacinamide, or
(xvi) 0.000-0.002 mg/L riboflavin, or
(xvii) 0.00-0.01 mg/L thiamine, or
(xviii) 0.00-0.01 mg/L pyridoxine, or
(xix) 0-2 µg/L biotin, or
(xx) 0.00-0.05 µg/L vitamin B12, or
(xxi) 0.00-0.01 mg/L p-aminobenzoic acid, or
(xxii) 0-2 µg/L D-pantothenic acid, or
(xxiii) 0.00-0.03 mg/L choline chloride, or
(xxiv) 0.00-0.07 mg/L myo-inositol, or
(xxv) 0.0-0.1 mg/L insulin, or
(xxvi) 0.00-0.05 mg/L transferrin, or
(xxvii) 0.000-0.158 µg/L selenium, or
(xxviii) 0-10 weight-%, such as 0-5 weight-%, preferably 0-1 weight-%, most preferably 0.0-0.2 weight-%, blood serum, relative to the total weight of the storage medium, or
(xxix) one or more than one or all of (i)-(xxviii).

4. The use of claim 1 or claim 3, or the method of of claim 2 or claim 3, wherein the storage medium is a hypertonic, isotonic, or hypotonic, buffered or unbuffered solution, preferably an isotonic or hypertonic, more preferably isotonic, solution, and most preferably an isotonic phosphate-buffered solution.

5. The use or the method of claim 4, wherein the storage medium is an, optionally isotonic, solution that is buffered with alkali dihydrogenphosphate and dialkali hydrogenphosphate and preferably comprises dialkali hydrogenphosphate and alkali dihydrogenphosphate at a total amount of 10-50 mM, wherein the alkali is sodium, potassium or a mixture thereof, preferably sodium.

6. The use of any one of claims 1 and 3-5, or the method of any one of claims 2-5, wherein the storage medium comprises adenine, guanosine, glucose, and mannitol.

7. The use or the method of any one of claims 4-6, wherein the storage medium comprises:
- 0.5-4.0 mM, in particular 1.0-1.8 mM, more particularly 1.4 mM adenine,
- 0.5-4.0 mM, in particular 1.0-1.8 mM, more particularly 1.4 mM guanosine,
- 20-80 mM, in particular 45-65 mM, more particularly 47-52 mM glucose, and
- 20-80 mM, in particular 45-65 mM mM, more particularly 55 mM mannitol, and preferably comprises:
(i) 0.0-0.2 mg/L valine, or
(ii) 0.0-0.4 mg/L lysine, or
(iii) 0.0-0.5 mg/L leucine, or
(iv) 0.0-0.5 mg/L isoleucine, or
(v) 0.0-0.2 mg/L threonine, or
(vi) 0.0-0.1 mg/L phenylalanine, or
(vii) 0.00-0.05 mg/L tryptophan, or
(viii) 0.0-0.1 mg/L methionine, or
(ix) 0.0-0.1 mg/L histidine, or
(x) 0-3 mg/L glutamine, or
(xi) 0.0-0.6 mg/L cysteine, or
(xii) 0-2 mg/L arginine, or
(xiii) 0-1 mg/L tyrosine, or
(xiv) 0.00-0.01 mg/L folic acid, or
(xv) 0.00-0.01 mg/L niacinamide, or
(xvi) 0.000-0.002 mg/L riboflavin, or
(xvii) 0.00-0.01 mg/L thiamine, or
(xviii) 0.00-0.01 mg/L pyridoxine, or
(xix) 0-2 µg/L biotin, or
(xx) 0.00-0.05 µg/L vitamin B12, or
(xxi) 0.00-0.01 mg/L p-aminobenzoic acid, or
(xxii) 0-2 µg/L D-pantothenic acid, or
(xxiii) 0.00-0.03 mg/L choline chloride, or
(xxiv) 0.00-0.07 mg/L myo-inositol, or
(xxv) 0.0-0.1 mg/L insulin, or
(xxvi) 0.00-0.05 mg/L transferrin, or
(xxvii) 0.000-0.158 µg/L selenium, or
(xxviii) one or more than one or all of (i)-(xxvii), and
further preferably comprises 0-10 weight-%, such as 0-5 weight-%, particularly 0-1 weight-%, especially 0.0-0.2 weight-%, blood serum, relative to the total weight of the storage medium.

8. The use or the method of any one of claims 4-7, wherein the storage medium further comprises sodium chloride, preferably at an concentration of 40-110mM, more preferably 60-85 mM, and most preferably 73 mM.

9. The use of any one of claims 1 and 3-8, or the method of any one of claims 2-8, wherein the cells in storage medium are stored at a temperature in the range of from 0°C to 24°C, such as from 1°C to 20°C, preferably from 1°C to 15°C, more preferably from 1°C to 10°C, even more preferably from 2°C to 8°C, most preferably at 4±2°C.

10. The use of any one of claims 1 and 3-9, or the method of any one of claims 2-9, wherein the sericin comprises at least one region having the amino acid sequence of SEQ ID NO:1.

11. The use of any one of claims 1 and 3-10, or the method of any one of claims 2-10, wherein the sericin is selected from:
- the sericin having Sigma-Aldrich product number S5201,
- the sericin of CAS number 474338-68-6,
- the sericin isolated from *Bombyx mori* silk fibers as described in Terada et al, Cytotechnology 2002, 40:3-12, and
- naturally derived sericins and sericin derivatives as well as mixtures thereof which comprise an amino acid sequence having having at least 60% identity with an amino acid sequence selected from SEQ ID NOs:1-10, and which have at least 60% of the activity in reducing storage-associated cell death exhibited by the sericin having Sigma-Aldrich product number S5201 or the sericin of CAS number 474338-68-6 or the sericin isolated from *Bombyx mori* silk fibers as described in Terada et al, Cytotechnology 2002, 40:3-12,
wherein, optionally, the activity in reducing storage-associated cell death is determined by the method comprising the following steps:
(1) transferring a cell population selected from human mononuclear leukocytes, human CD3+ T lymphocytes, human CD14+ monocytes and human CD34+ stem cells into a storage medium so as to prepare a suspension of 2x10⁵ cells / mL;
(2) storing the cell suspension in a closed container for 1 week at 4°C;
(3) then mixing the cell suspension with a dead cell stain;
(4) measuring the number of non-stained cells in the cell suspension using flow cytometry;
wherein steps (1)-(4) are performed for each of the following storage media:
(i) 1.432 g/L Na₂HPO₄ · 2 H₂O, 1.255 g/L NaH₂PO₄ · 2 H₂O, 0.194 g/L adenine, 9.405 g/L glucose monohydrate, 0.408 g/L guanosine, 4.210 g/L NaCl, 10.000 g/L mannitol, in water (PAGGS-M),
(ii) PAGGS-M supplemented with 0.5 weight-% of the tested sericin, relative to the total weight of PAGGS-M plus sericin (PAGGS-M+sericin);
and wherein the activity in reducing storage-associated cell death corresponds to the difference of the number of non-stained cells after storage in PAGGS-M+sericin minus the number of non-stained cells after storage in PAGGS-M.

12. The use of any one of claims 1 and 3-11, or the method of any one of claims 2-11, wherein the leukocytes are selected from T lymphocytes, B lymphocytes, NK cells, monocytes, dendritic cells and granulocytes, and preferably are T lymphocytes or monocytes, most preferably CD3 positive T lymphocytes or CD14 positive monocytes;
wherein the stem cells are hematopoietic stem cells, preferably CD34 positive hematopoietic stem cells.

13. A medium for storing cells selected from leukocytes and stem cells, wherein the medium is an aqueous solution comprising sericin and:
(i) 0.0-0.2 mg/L valine, or
(ii) 0.0-0.4 mg/L lysine, or
(iii) 0.0-0.5 mg/L leucine, or
(iv) 0.0-0.5 mg/L isoleucine, or
(v) 0.0-0.2 mg/L threonine, or
(vi) 0.0-0.1 mg/L phenylalanine, or
(vii) 0.00-0.05 mg/L tryptophan, or
(viii) 0.0-0.1 mg/L methionine, or
(ix) 0.0-0.1 mg/L histidine, or
(x) 0-3 mg/L glutamine, or
(xi) 0.0-0.6 mg/L cysteine, or
(xii) 0-2 mg/L arginine, or
(xiii) 0-1 mg/L tyrosine, or
(xiv) 0.00-0.01 mg/L folic acid, or
(xv) 0.00-0.01 mg/L niacinamide, or
(xvi) 0.000-0.002 mg/L riboflavin, or
(xvii) 0.00-0.01 mg/L thiamine, or
(xviii) 0.00-0.01 mg/L pyridoxine, or
(xix) 0-2 µg/L biotin, or
(xx) 0.00-0.05 µg/L vitamin B12, or
(xxi) 0.00-0.01 mg/L p-aminobenzoic acid, or
(xxii) 0-2 µg/L D-pantothenic acid, or
(xxiii) 0.00-0.03 mg/L choline chloride, or
(xxiv) 0.00-0.07 mg/L myo-inositol, or
(xxv) 0.0-0.1 mg/L insulin, or
(xxvi) 0.00-0.05 mg/L transferrin, or
(xxvii) 0.000-0.158 µg/L selenium, or
(xxviii) one or more than one or all of (i)-(xxvii);
and wherein, optionally,
(1) the medium is a medium as defined in any one of claims 3-8, or
(2) the sericin is a sericin as defined in claim 10 or claim 11, or
(3) both or (1) and (2).

14. A dry medium formulation or a medium concentrate that can be reconstituted with a solvent, preferably water, so as to form a medium as defined in any one of claims 1-13.

15. A cell suspension in a medium as defined in any one of claims 1-13 having a cell density of 0.001 to 2·10⁸ cells/mL, wherein the cells are selected from leukocytes and stem cells, preferably of human origin, and, optionally, are further defined as in claim 12.


SEQUENCE LISTING

<110>  Friedrich-Alexander-Universität Erlangen-Nürnberg

<120>  Medium additive for reducing storage-associated death of
       leukocytes and stem cells

<130>  EPA-149 906

<160>  10

<170>  PatentIn version 3.5

<210>  1
<211>  38
<212>  PRT
<213>  Artificial Sequence

<220>
<223>  artificial sequence derived from Bombyx mori sericin

<400>  1

Ser Ser Thr Gly Ser Ser Ser Asn Thr Asp Ser Asn Ser Asn Ser Ala
1               5                   10                  15

Gly Ser Ser Thr Ser Gly Gly Ser Ser Thr Tyr Gly Tyr Ser Ser Asn
            20                  25                  30

Ser Arg Asp Gly Ser Val
        35

<210>  2
<211>  1217
<212>  PRT
<213>  Bombyx mori

<400>  2

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr

                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Thr Gly
    450                 455                 460

Glu Glu Glu Glu Leu Phe Asp Val Val Ser Tyr Gln Lys Ile Glu Asp
465                 470                 475                 480

Gly Lys Pro Val Ile Ile Met Lys Val Ile Pro Val Glu Lys Ser Ala
                485                 490                 495

Ser Gln Ser Ser Ser Ser Arg Ser Ser Gln Glu Ser Ala Ser Tyr Ser
            500                 505                 510

Ser Ser Ser Ser Ser Ser Thr Leu Ser Glu Asp Ser Ser Glu Val Asp
        515                 520                 525

Ile Asp Leu Gly Asn Leu Gly Trp Trp Trp Asn Ser Asp Asn Lys Ala
    530                 535                 540

Gln Arg Ala Ala Gly Gly Ala Thr Lys Ser Glu Ala Ser Ser Ser Thr
545                 550                 555                 560

Gln Ala Thr Thr Val Ser Gly Ala Asp Asp Ser Ala Asp Ser Tyr Thr
                565                 570                 575

Trp Trp Trp Asn Pro Arg Arg Ser Ser Ser Ser Ser Ser Ser Ala Ser
            580                 585                 590

Ser Ser Ser Ser Gly Ser Asn Val Gly Gly Ser Ser Gln Ser Ser Gly
        595                 600                 605

Ser Ser Thr Ser Gly Ser Asn Ala Arg Gly His Leu Gly Thr Val Ser
    610                 615                 620

Ser Thr Gly Ser Thr Ser Asn Thr Asp Ser Ser Ser Lys Ser Ala Gly
625                 630                 635                 640

Ser Arg Thr Ser Gly Gly Ser Ser Thr Tyr Gly Tyr Ser Ser Ser His
                645                 650                 655

Arg Gly Gly Ser Val Ser Ser Thr Gly Ser Ser Ser Asn Thr Asp Ser
            660                 665                 670

Ser Thr Lys Asn Ala Gly Ser Ser Thr Ser Gly Gly Ser Ser Thr Tyr
        675                 680                 685

Gly Tyr Ser Ser Ser His Arg Gly Gly Ser Val Ser Ser Thr Gly Ser
    690                 695                 700

Ser Ser Asn Thr Asp Ser Ser Thr Lys Asn Ala Gly Ser Ser Thr Ser
705                 710                 715                 720

Gly Gly Ser Ser Thr Tyr Gly Tyr Ser Ser Ser His Arg Gly Gly Ser
                725                 730                 735

Val Ser Ser Thr Gly Ser Ser Ser Asn Thr Asp Ser Ser Thr Lys Ser
            740                 745                 750

Ala Gly Ser Ser Thr Ser Gly Gly Ser Ser Thr Tyr Gly Tyr Ser Ser
        755                 760                 765

Arg His Arg Gly Gly Arg Val Ser Ser Thr Gly Ser Ser Ser Thr Thr
    770                 775                 780

Asp Ala Ser Ser Asn Ser Val Gly Ser Ser Thr Ser Gly Gly Ser Ser
785                 790                 795                 800

Thr Tyr Gly Tyr Ser Ser Asn Ser Arg Asp Gly Ser Val Ser Ser Thr
                805                 810                 815

Gly Ser Ser Ser Asn Thr Asp Ser Asn Ser Asn Ser Ala Gly Ser Ser
            820                 825                 830

Thr Ser Gly Gly Ser Ser Thr Tyr Gly Tyr Ser Ser Asn Ser Arg Asp
        835                 840                 845

Gly Ser Val Ser Ser Thr Gly Ser Ser Ser Asn Thr Asp Ser Asn Ser
    850                 855                 860

Asn Ser Ala Gly Ser Ser Thr Ser Gly Gly Ser Ser Thr Tyr Gly Tyr
865                 870                 875                 880

Ser Ser Asn Ser Arg Asp Gly Ser Val Ser Ser Thr Gly Ser Ser Ser
                885                 890                 895

Asn Thr Asp Ala Ser Thr Asp Leu Thr Gly Ser Ser Thr Ser Gly Gly
            900                 905                 910

Ser Ser Thr Tyr Gly Tyr Ser Ser Asp Ser Arg Asp Gly Ser Val Ser
        915                 920                 925

Ser Thr Gly Ser Ser Ser Asn Thr Asp Ala Ser Thr Asp Leu Ala Gly
    930                 935                 940

Ser Ser Thr Ser Gly Gly Ser Ser Thr Tyr Gly Tyr Ser Ser Asp Cys
945                 950                 955                 960

Gly Asp Gly Ser Val Ser Ser Thr Gly Ser Ser Ser Asn Thr Asp Ala
                965                 970                 975

Ser Thr Asp Leu Ala Gly Ser Ser Thr Ser Gly Gly Ser Ser Thr Tyr
            980                 985                 990

Gly Tyr Ser Ser Asn Ser Arg Asp  Gly Ser Val Ser Ser  Thr Gly Ser
        995                 1000                1005

Ser Ser  Asn Thr Asp Ala Ser  Thr Asp Leu Thr Gly  Ser Ser Thr
    1010                1015                1020

Ser Gly  Gly Ser Ser Thr Tyr  Gly Tyr Ser Ser Ser  Asn Arg Asp
    1025                1030                1035

Gly Ser  Val Leu Ala Thr Gly  Ser Ser Ser Asn Thr  Asp Ala Ser
    1040                1045                1050

Thr Thr  Glu Glu Ser Thr Thr  Ser Ala Gly Ser Ser  Thr Glu Gly
    1055                1060                1065

Tyr Ser  Ser Ser Ser His Asp  Gly Ser Val Thr Ser  Thr Asp Gly
    1070                1075                1080

Ser Ser  Thr Ser Gly Gly Ala  Ser Ser Ser Ser Ala  Ser Thr Ala

1085                    1090                    1095

Lys Ser  Asp Ala Ala Ser Ser  Glu Asp Gly Phe Trp  Trp Trp Asn
    1100                    1105                    1110

Arg Arg  Lys Ser Gly Ser Gly  His Lys Ser Ala Thr  Val Gln Ser
    1115                    1120                    1125

Ser Thr  Thr Asp Lys Thr Ser  Thr Asp Ser Ala Ser  Ser Thr Asp
    1130                    1135                    1140

Ser Thr  Ser Ser Thr Ser Gly  Ala Ser Thr Thr Thr  Ser Gly Ser
    1145                    1150                    1155

Ser Ser  Thr Ser Gly Gly Ser  Ser Thr Ser Asp Ala  Ser Ser Thr
    1160                    1165                    1170

Ser Ser  Ser Val Ser Arg Ser  His Arg Ser Gly Val  Asn Arg Leu
    1175                    1180                    1185

Leu His  Lys Pro Gly Gln Gly  Lys Ile Cys Leu Cys  Phe Glu Asn
    1190                    1195                    1200

Ile Phe  Asp Ile Pro Tyr His  Leu Arg Lys Asn Ile  Gly Val
    1205                    1210                    1215

<210>  3
<211>  1269
<212>  PRT
<213>  Bombyx mori

<400>  3

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr

                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Thr Gly
    450                 455                 460

Glu Glu Glu Glu Leu Phe Asp Val Val Ser Tyr Gln Lys Ile Glu Asp
465                 470                 475                 480

Gly Lys Pro Val Ile Ile Met Lys Val Ile Pro Val Glu Lys Ser Ala
                485                 490                 495

Ser Gln Ser Ser Ser Ser Arg Ser Ser Gln Glu Ser Ala Ser Tyr Ser
            500                 505                 510

Ser Ser Ser Ser Ser Ser Ser Pro Arg Pro Leu Ser Ile Trp Thr Gly
        515                 520                 525

Pro Gly Arg Asp Ile Pro Phe Phe Arg Arg Lys Tyr Ser Gly Ala Arg
    530                 535                 540

Ser Val Pro Lys Pro Ile Pro Ile Pro Ile Gln Ser Lys Gly Ser Thr
545                 550                 555                 560

Glu Arg Pro Gln Pro Thr Gly His Arg Ser Ala Leu Ser Glu Asp Ser
                565                 570                 575

Ser Glu Val Asp Ile Asp Leu Gly Asn Leu Gly Trp Trp Trp Asn Ser
            580                 585                 590

Asp Asn Lys Ala Gln Arg Ala Ala Gly Gly Ala Thr Lys Ser Glu Ala
        595                 600                 605

Ser Ser Ser Thr Gln Ala Thr Thr Val Ser Gly Ala Asp Asp Ser Ala
    610                 615                 620

Asp Ser Tyr Thr Trp Trp Trp Asn Pro Arg Arg Ser Ser Ser Ser Ser
625                 630                 635                 640

Ser Ser Ala Ser Ser Ser Ser Ser Gly Ser Asn Val Gly Gly Ser Ser
                645                 650                 655

Gln Ser Ser Gly Ser Ser Thr Ser Gly Ser Asn Ala Arg Gly His Leu
            660                 665                 670

Gly Thr Val Ser Ser Thr Gly Ser Thr Ser Asn Thr Asp Ser Ser Ser
        675                 680                 685

Lys Ser Ala Gly Ser Arg Thr Ser Gly Gly Ser Ser Thr Tyr Gly Tyr
    690                 695                 700

Ser Ser Ser His Arg Gly Gly Ser Val Ser Ser Thr Gly Ser Ser Ser
705                 710                 715                 720

Asn Thr Asp Ser Ser Thr Lys Asn Ala Gly Ser Ser Thr Ser Gly Gly
                725                 730                 735

Ser Ser Thr Tyr Gly Tyr Ser Ser Ser His Arg Gly Gly Ser Val Ser
            740                 745                 750

Ser Thr Gly Ser Ser Ser Asn Thr Asp Ser Ser Thr Lys Asn Ala Gly
        755                 760                 765

Ser Ser Thr Ser Gly Gly Ser Ser Thr Tyr Gly Tyr Ser Ser Ser His
    770                 775                 780

Arg Gly Gly Ser Val Ser Ser Thr Gly Ser Ser Ser Asn Thr Asp Ser
785                 790                 795                 800

Ser Thr Lys Ser Ala Gly Ser Ser Thr Ser Gly Gly Ser Ser Thr Tyr
                805                 810                 815

Gly Tyr Ser Ser Arg His Arg Gly Gly Arg Val Ser Ser Thr Gly Ser
            820                 825                 830

Ser Ser Thr Thr Asp Ala Ser Ser Asn Ser Val Gly Ser Ser Thr Ser
        835                 840                 845

Gly Gly Ser Ser Thr Tyr Gly Tyr Ser Ser Asn Ser Arg Asp Gly Ser
    850                 855                 860

Val Ser Ser Thr Gly Ser Ser Ser Asn Thr Asp Ser Asn Ser Asn Ser
865                 870                 875                 880

Ala Gly Ser Ser Thr Ser Gly Gly Ser Ser Thr Tyr Gly Tyr Ser Ser
                885                 890                 895

Asn Ser Arg Asp Gly Ser Val Ser Ser Thr Gly Ser Ser Ser Asn Thr
            900                 905                 910

Asp Ser Asn Ser Asn Ser Ala Gly Ser Ser Thr Ser Gly Gly Ser Ser
        915                 920                 925

Thr Tyr Gly Tyr Ser Ser Asn Ser Arg Asp Gly Ser Val Ser Ser Thr
    930                 935                 940

Gly Ser Ser Ser Asn Thr Asp Ala Ser Thr Asp Leu Thr Gly Ser Ser
945                 950                 955                 960

Thr Ser Gly Gly Ser Ser Thr Tyr Gly Tyr Ser Ser Asp Ser Arg Asp
                965                 970                 975

Gly Ser Val Ser Ser Thr Gly Ser Ser Ser Asn Thr Asp Ala Ser Thr
            980                 985                 990

Asp Leu Ala Gly Ser Ser Thr Ser  Gly Gly Ser Ser Thr  Tyr Gly Tyr
        995                 1000                1005

Ser Ser  Asp Cys Gly Asp Gly  Ser Val Ser Ser Thr  Gly Ser Ser
    1010                1015                1020

Ser Asn  Thr Asp Ala Ser Thr  Asp Leu Ala Gly Ser  Ser Thr Ser
    1025                1030                1035

Gly Gly  Ser Ser Thr Tyr Gly  Tyr Ser Ser Asn Ser  Arg Asp Gly
    1040                1045                1050

Ser Val  Ser Ser Thr Gly Ser  Ser Ser Asn Thr Asp  Ala Ser Thr
    1055                1060                1065

Asp Leu  Thr Gly Ser Ser Thr  Ser Gly Gly Ser Ser  Thr Tyr Gly
    1070                1075                1080

Tyr Ser  Ser Ser Asn Arg Asp  Gly Ser Val Leu Ala  Thr Gly Ser

1085                1090                1095

Ser Ser  Asn Thr Asp Ala Ser  Thr Thr Glu Glu Ser  Thr Thr Ser
    1100                1105                1110

Ala Gly  Ser Ser Thr Glu Gly  Tyr Ser Ser Ser Ser  His Asp Gly
    1115                1120                1125

Ser Val  Thr Ser Thr Asp Gly  Ser Ser Thr Ser Gly  Gly Ala Ser
    1130                1135                1140

Ser Ser  Ser Ala Ser Thr Ala  Lys Ser Asp Ala Ala  Ser Ser Glu
    1145                1150                1155

Asp Gly  Phe Trp Trp Trp Asn  Arg Arg Lys Ser Gly  Ser Gly His
    1160                1165                1170

Lys Ser  Ala Thr Val Gln Ser  Ser Thr Thr Asp Lys  Thr Ser Thr
    1175                1180                1185

Asp Ser  Ala Ser Ser Thr Asp  Ser Thr Ser Ser Thr  Ser Gly Ala
    1190                1195                1200

Ser Thr  Thr Thr Ser Gly Ser  Ser Ser Thr Ser Gly  Gly Ser Ser
    1205                1210                1215

Thr Ser  Asp Ala Ser Ser Thr  Ser Ser Ser Val Ser  Arg Ser His
    1220                1225                1230

Arg Ser  Gly Val Asn Arg Leu  Leu His Lys Pro Gly  Gln Gly Lys
    1235                1240                1245

Ile Cys  Leu Cys Phe Glu Asn  Ile Phe Asp Ile Pro  Tyr His Leu
    1250                1255                1260

Arg Lys  Asn Ile Gly Val
    1265

<210>  4
<211>  1243
<212>  PRT
<213>  Bombyx mori

<400>  4

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val

20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr
                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Thr Gly
    450                 455                 460

Glu Glu Glu Glu Leu Phe Asp Val Val Ser Tyr Gln Lys Ile Glu Asp
465                 470                 475                 480

Gly Lys Pro Val Ile Ile Met Lys Val Ile Pro Val Gly Pro Arg Pro
                485                 490                 495

Leu Ser Ile Trp Thr Gly Pro Gly Arg Asp Ile Pro Phe Phe Arg Arg
            500                 505                 510

Lys Tyr Ser Gly Ala Arg Ser Val Pro Lys Pro Ile Pro Ile Pro Ile
        515                 520                 525

Gln Ser Lys Gly Ser Thr Glu Arg Pro Gln Pro Thr Gly His Arg Ser
    530                 535                 540

Ala Leu Ser Glu Asp Ser Ser Glu Val Asp Ile Asp Leu Gly Asn Leu
545                 550                 555                 560

Gly Trp Trp Trp Asn Ser Asp Asn Lys Ala Gln Arg Ala Ala Gly Gly
                565                 570                 575

Ala Thr Lys Ser Glu Ala Ser Ser Ser Thr Gln Ala Thr Thr Val Ser
            580                 585                 590

Gly Ala Asp Asp Ser Ala Asp Ser Tyr Thr Trp Trp Trp Asn Pro Arg
        595                 600                 605

Arg Ser Ser Ser Ser Ser Ser Ser Ala Ser Ser Ser Ser Ser Gly Ser
    610                 615                 620

Asn Val Gly Gly Ser Ser Gln Ser Ser Gly Ser Ser Thr Ser Gly Ser
625                 630                 635                 640

Asn Ala Arg Gly His Leu Gly Thr Val Ser Ser Thr Gly Ser Thr Ser
                645                 650                 655

Asn Thr Asp Ser Ser Ser Lys Ser Ala Gly Ser Arg Thr Ser Gly Gly
            660                 665                 670

Ser Ser Thr Tyr Gly Tyr Ser Ser Ser His Arg Gly Gly Ser Val Ser
        675                 680                 685

Ser Thr Gly Ser Ser Ser Asn Thr Asp Ser Ser Thr Lys Asn Ala Gly
    690                 695                 700

Ser Ser Thr Ser Gly Gly Ser Ser Thr Tyr Gly Tyr Ser Ser Ser His
705                 710                 715                 720

Arg Gly Gly Ser Val Ser Ser Thr Gly Ser Ser Ser Asn Thr Asp Ser
                725                 730                 735

Ser Thr Lys Asn Ala Gly Ser Ser Thr Ser Gly Gly Ser Ser Thr Tyr
            740                 745                 750

Gly Tyr Ser Ser Ser His Arg Gly Gly Ser Val Ser Ser Thr Gly Ser
        755                 760                 765

Ser Ser Asn Thr Asp Ser Ser Thr Lys Ser Ala Gly Ser Ser Thr Ser
    770                 775                 780

Gly Gly Ser Ser Thr Tyr Gly Tyr Ser Ser Arg His Arg Gly Gly Arg
785                 790                 795                 800

Val Ser Ser Thr Gly Ser Ser Ser Thr Thr Asp Ala Ser Ser Asn Ser
                805                 810                 815

Val Gly Ser Ser Thr Ser Gly Gly Ser Ser Thr Tyr Gly Tyr Ser Ser
            820                 825                 830

Asn Ser Arg Asp Gly Ser Val Ser Ser Thr Gly Ser Ser Ser Asn Thr
        835                 840                 845

Asp Ser Asn Ser Asn Ser Ala Gly Ser Ser Thr Ser Gly Gly Ser Ser
    850                 855                 860

Thr Tyr Gly Tyr Ser Ser Asn Ser Arg Asp Gly Ser Val Ser Ser Thr
865                 870                 875                 880

Gly Ser Ser Ser Asn Thr Asp Ser Asn Ser Asn Ser Ala Gly Ser Ser
                885                 890                 895

Thr Ser Gly Gly Ser Ser Thr Tyr Gly Tyr Ser Ser Asn Ser Arg Asp
            900                 905                 910

Gly Ser Val Ser Ser Thr Gly Ser Ser Ser Asn Thr Asp Ala Ser Thr
        915                 920                 925

Asp Leu Thr Gly Ser Ser Thr Ser Gly Gly Ser Ser Thr Tyr Gly Tyr
    930                 935                 940

Ser Ser Asp Ser Arg Asp Gly Ser Val Ser Ser Thr Gly Ser Ser Ser
945                 950                 955                 960

Asn Thr Asp Ala Ser Thr Asp Leu Ala Gly Ser Ser Thr Ser Gly Gly
                965                 970                 975

Ser Ser Thr Tyr Gly Tyr Ser Ser Asp Cys Gly Asp Gly Ser Val Ser
            980                 985                 990

Ser Thr Gly Ser Ser Ser Asn Thr  Asp Ala Ser Thr Asp  Leu Ala Gly
        995                 1000                1005

Ser Ser  Thr Ser Gly Gly Ser  Ser Thr Tyr Gly Tyr  Ser Ser Asn
    1010                1015                1020

Ser Arg  Asp Gly Ser Val Ser  Ser Thr Gly Ser Ser  Ser Asn Thr

1025                    1030                    1035

Asp Ala  Ser Thr Asp Leu Thr  Gly Ser Ser Thr Ser  Gly Gly Ser
    1040                    1045                    1050

Ser Thr  Tyr Gly Tyr Ser Ser  Ser Asn Arg Asp Gly  Ser Val Leu
    1055                    1060                    1065

Ala Thr  Gly Ser Ser Ser Asn  Thr Asp Ala Ser Thr  Thr Glu Glu
    1070                    1075                    1080

Ser Thr  Thr Ser Ala Gly Ser  Ser Thr Glu Gly Tyr  Ser Ser Ser
    1085                    1090                    1095

Ser His  Asp Gly Ser Val Thr  Ser Thr Asp Gly Ser  Ser Thr Ser
    1100                    1105                    1110

Gly Gly  Ala Ser Ser Ser Ser  Ala Ser Thr Ala Lys  Ser Asp Ala
    1115                    1120                    1125

Ala Ser  Ser Glu Asp Gly Phe  Trp Trp Trp Asn Arg  Arg Lys Ser
    1130                    1135                    1140

Gly Ser  Gly His Lys Ser Ala  Thr Val Gln Ser Ser  Thr Thr Asp
    1145                    1150                    1155

Lys Thr  Ser Thr Asp Ser Ala  Ser Ser Thr Asp Ser  Thr Ser Ser
    1160                    1165                    1170

Thr Ser  Gly Ala Ser Thr Thr  Thr Ser Gly Ser Ser  Ser Thr Ser
    1175                    1180                    1185

Gly Gly  Ser Ser Thr Ser Asp  Ala Ser Ser Thr Ser  Ser Ser Val
    1190                    1195                    1200

Ser Arg  Ser His Arg Ser Gly  Val Asn Arg Leu Leu  His Lys Pro
    1205                    1210                    1215

Gly Gln  Gly Lys Ile Cys Leu  Cys Phe Glu Asn Ile  Phe Asp Ile
    1220                    1225                    1230

Pro Tyr  His Leu Arg Lys Asn  Ile Gly Val
    1235                    1240

<210>  5
<211>  1238
<212>  PRT
<213>  Bombyx mori

<400>  5

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr
                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Lys Ser
    450                 455                 460

Ala Ser Gln Ser Ser Ser Ser Arg Ser Ser Gln Glu Ser Ala Ser Tyr
465                 470                 475                 480

Ser Ser Ser Ser Ser Ser Ser Ser Pro Arg Pro Leu Ser Ile Trp Thr
                485                 490                 495

Gly Pro Gly Arg Asp Ile Pro Phe Phe Arg Arg Lys Tyr Ser Gly Ala
            500                 505                 510

Arg Ser Val Pro Lys Pro Ile Pro Ile Pro Ile Gln Ser Lys Gly Ser
        515                 520                 525

Thr Glu Arg Pro Gln Pro Thr Gly His Arg Ser Ala Leu Ser Glu Asp
    530                 535                 540

Ser Ser Glu Val Asp Ile Asp Leu Gly Asn Leu Gly Trp Trp Trp Asn
545                 550                 555                 560

Ser Asp Asn Lys Ala Gln Arg Ala Ala Gly Gly Ala Thr Lys Ser Glu
                565                 570                 575

Ala Ser Ser Ser Thr Gln Ala Thr Thr Val Ser Gly Ala Asp Asp Ser
            580                 585                 590

Ala Asp Ser Tyr Thr Trp Trp Trp Asn Pro Arg Arg Ser Ser Ser Ser
        595                 600                 605

Ser Ser Ser Ala Ser Ser Ser Ser Ser Gly Ser Asn Val Gly Gly Ser
    610                 615                 620

Ser Gln Ser Ser Gly Ser Ser Thr Ser Gly Ser Asn Ala Arg Gly His
625                 630                 635                 640

Leu Gly Thr Val Ser Ser Thr Gly Ser Thr Ser Asn Thr Asp Ser Ser
                645                 650                 655

Ser Lys Ser Ala Gly Ser Arg Thr Ser Gly Gly Ser Ser Thr Tyr Gly
            660                 665                 670

Tyr Ser Ser Ser His Arg Gly Gly Ser Val Ser Ser Thr Gly Ser Ser
        675                 680                 685

Ser Asn Thr Asp Ser Ser Thr Lys Asn Ala Gly Ser Ser Thr Ser Gly
    690                 695                 700

Gly Ser Ser Thr Tyr Gly Tyr Ser Ser Ser His Arg Gly Gly Ser Val
705                 710                 715                 720

Ser Ser Thr Gly Ser Ser Ser Asn Thr Asp Ser Ser Thr Lys Asn Ala
                725                 730                 735

Gly Ser Ser Thr Ser Gly Gly Ser Ser Thr Tyr Gly Tyr Ser Ser Ser
            740                 745                 750

His Arg Gly Gly Ser Val Ser Ser Thr Gly Ser Ser Ser Asn Thr Asp
        755                 760                 765

Ser Ser Thr Lys Ser Ala Gly Ser Ser Thr Ser Gly Gly Ser Ser Thr
    770                 775                 780

Tyr Gly Tyr Ser Ser Arg His Arg Gly Gly Arg Val Ser Ser Thr Gly
785                 790                 795                 800

Ser Ser Ser Thr Thr Asp Ala Ser Ser Asn Ser Val Gly Ser Ser Thr
                805                 810                 815

Ser Gly Gly Ser Ser Thr Tyr Gly Tyr Ser Ser Asn Ser Arg Asp Gly
            820                 825                 830

Ser Val Ser Ser Thr Gly Ser Ser Ser Asn Thr Asp Ser Asn Ser Asn
        835                 840                 845

Ser Ala Gly Ser Ser Thr Ser Gly Gly Ser Ser Thr Tyr Gly Tyr Ser
    850                 855                 860

Ser Asn Ser Arg Asp Gly Ser Val Ser Ser Thr Gly Ser Ser Ser Asn
865                 870                 875                 880

Thr Asp Ser Asn Ser Asn Ser Ala Gly Ser Ser Thr Ser Gly Gly Ser
                885                 890                 895

Ser Thr Tyr Gly Tyr Ser Ser Asn Ser Arg Asp Gly Ser Val Ser Ser
            900                 905                 910

Thr Gly Ser Ser Ser Asn Thr Asp Ala Ser Thr Asp Leu Thr Gly Ser
        915                 920                 925

Ser Thr Ser Gly Gly Ser Ser Thr Tyr Gly Tyr Ser Ser Asp Ser Arg
    930                 935                 940

Asp Gly Ser Val Ser Ser Thr Gly Ser Ser Ser Asn Thr Asp Ala Ser
945                 950                 955                 960

Thr Asp Leu Ala Gly Ser Ser Thr Ser Gly Gly Ser Ser Thr Tyr Gly
                965                 970                 975

Tyr Ser Ser Asp Cys Gly Asp Gly Ser Val Ser Ser Thr Gly Ser Ser
            980                 985                 990

Ser Asn Thr Asp Ala Ser Thr Asp  Leu Ala Gly Ser Ser  Thr Ser Gly

995                 1000                1005

Gly Ser Ser Thr Tyr Gly Tyr Ser Ser Asn Ser Arg Asp Gly Ser
    1010                1015                1020

Val Ser Ser Thr Gly Ser Ser Ser Asn Thr Asp Ala Ser Thr Asp
    1025                1030                1035

Leu Thr Gly Ser Ser Thr Ser Gly Gly Ser Ser Thr Tyr Gly Tyr
    1040                1045                1050

Ser Ser Ser Asn Arg Asp Gly Ser Val Leu Ala Thr Gly Ser Ser
    1055                1060                1065

Ser Asn Thr Asp Ala Ser Thr Thr Glu Glu Ser Thr Thr Ser Ala
    1070                1075                1080

Gly Ser Ser Thr Glu Gly Tyr Ser Ser Ser Ser His Asp Gly Ser
    1085                1090                1095

Val Thr Ser Thr Asp Gly Ser Ser Thr Ser Gly Gly Ala Ser Ser
    1100                1105                1110

Ser Ser Ala Ser Thr Ala Lys Ser Asp Ala Ala Ser Ser Glu Asp
    1115                1120                1125

Gly Phe Trp Trp Trp Asn Arg Arg Lys Ser Gly Ser Gly His Lys
    1130                1135                1140

Ser Ala Thr Val Gln Ser Ser Thr Thr Asp Lys Thr Ser Thr Asp
    1145                1150                1155

Ser Ala Ser Ser Thr Asp Ser Thr Ser Ser Thr Ser Gly Ala Ser
    1160                1165                1170

Thr Thr Thr Ser Gly Ser Ser Ser Thr Ser Gly Gly Ser Ser Thr
    1175                1180                1185

Ser Asp Ala Ser Ser Thr Ser Ser Ser Val Ser Arg Ser His Arg
    1190                1195                1200

Ser Gly Val Asn Arg Leu Leu His Lys Pro Gly Gln Gly Lys Ile
    1205                1210                1215

Cys Leu Cys Phe Glu Asn Ile Phe Asp Ile Pro Tyr His Leu Arg
    1220                1225                1230

Lys Asn  Ile Gly Val
    1235

<210>  6
<211>  1191
<212>  PRT
<213>  Bombyx mori

<400>  6

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr
                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Thr Gly
    450                 455                 460

Glu Glu Glu Glu Leu Phe Asp Val Val Ser Tyr Gln Lys Ile Glu Asp
465                 470                 475                 480

Gly Lys Pro Val Ile Ile Met Lys Val Ile Pro Val Ala Leu Ser Glu
                485                 490                 495

Asp Ser Ser Glu Val Asp Ile Asp Leu Gly Asn Leu Gly Trp Trp Trp
            500                 505                 510

Asn Ser Asp Asn Lys Ala Gln Arg Ala Ala Gly Gly Ala Thr Lys Ser
        515                 520                 525

Glu Ala Ser Ser Ser Thr Gln Ala Thr Thr Val Ser Gly Ala Asp Asp
    530                 535                 540

Ser Ala Asp Ser Tyr Thr Trp Trp Trp Asn Pro Arg Arg Ser Ser Ser
545                 550                 555                 560

Ser Ser Ser Ser Ala Ser Ser Ser Ser Ser Gly Ser Asn Val Gly Gly
                565                 570                 575

Ser Ser Gln Ser Ser Gly Ser Ser Thr Ser Gly Ser Asn Ala Arg Gly
            580                 585                 590

His Leu Gly Thr Val Ser Ser Thr Gly Ser Thr Ser Asn Thr Asp Ser
        595                 600                 605

Ser Ser Lys Ser Ala Gly Ser Arg Thr Ser Gly Gly Ser Ser Thr Tyr
    610                 615                 620

Gly Tyr Ser Ser Ser His Arg Gly Gly Ser Val Ser Ser Thr Gly Ser
625                 630                 635                 640

Ser Ser Asn Thr Asp Ser Ser Thr Lys Asn Ala Gly Ser Ser Thr Ser
                645                 650                 655

Gly Gly Ser Ser Thr Tyr Gly Tyr Ser Ser Ser His Arg Gly Gly Ser
            660                 665                 670

Val Ser Ser Thr Gly Ser Ser Ser Asn Thr Asp Ser Ser Thr Lys Asn
        675                 680                 685

Ala Gly Ser Ser Thr Ser Gly Gly Ser Ser Thr Tyr Gly Tyr Ser Ser
    690                 695                 700

Ser His Arg Gly Gly Ser Val Ser Ser Thr Gly Ser Ser Ser Asn Thr
705                 710                 715                 720

Asp Ser Ser Thr Lys Ser Ala Gly Ser Ser Thr Ser Gly Gly Ser Ser
                725                 730                 735

Thr Tyr Gly Tyr Ser Ser Arg His Arg Gly Gly Arg Val Ser Ser Thr
            740                 745                 750

Gly Ser Ser Ser Thr Thr Asp Ala Ser Ser Asn Ser Val Gly Ser Ser
        755                 760                 765

Thr Ser Gly Gly Ser Ser Thr Tyr Gly Tyr Ser Ser Asn Ser Arg Asp
    770                 775                 780

Gly Ser Val Ser Ser Thr Gly Ser Ser Ser Asn Thr Asp Ser Asn Ser
785                 790                 795                 800

Asn Ser Ala Gly Ser Ser Thr Ser Gly Gly Ser Ser Thr Tyr Gly Tyr
                805                 810                 815

Ser Ser Asn Ser Arg Asp Gly Ser Val Ser Ser Thr Gly Ser Ser Ser
            820                 825                 830

Asn Thr Asp Ser Asn Ser Asn Ser Ala Gly Ser Ser Thr Ser Gly Gly
        835                 840                 845

Ser Ser Thr Tyr Gly Tyr Ser Ser Asn Ser Arg Asp Gly Ser Val Ser
    850                 855                 860

Ser Thr Gly Ser Ser Ser Asn Thr Asp Ala Ser Thr Asp Leu Thr Gly
865                 870                 875                 880

Ser Ser Thr Ser Gly Gly Ser Ser Thr Tyr Gly Tyr Ser Ser Asp Ser
                885                 890                 895

Arg Asp Gly Ser Val Ser Ser Thr Gly Ser Ser Ser Asn Thr Asp Ala
            900                 905                 910

Ser Thr Asp Leu Ala Gly Ser Ser Thr Ser Gly Gly Ser Ser Thr Tyr
        915                 920                 925

Gly Tyr Ser Ser Asp Cys Gly Asp Gly Ser Val Ser Ser Thr Gly Ser
    930                 935                 940

Ser Ser Asn Thr Asp Ala Ser Thr Asp Leu Ala Gly Ser Ser Thr Ser
945                 950                 955                 960

Gly Gly Ser Ser Thr Tyr Gly Tyr Ser Ser Asn Ser Arg Asp Gly Ser

                965                 970                 975

Val Ser Ser Thr Gly Ser Ser Ser Asn Thr Asp Ala Ser Thr Asp Leu
            980                 985                 990

Thr Gly Ser Ser Thr Ser Gly Gly Ser Ser Thr Tyr Gly Tyr Ser Ser
        995                 1000                1005

Ser Asn Arg Asp Gly Ser Val Leu Ala Thr Gly Ser Ser Ser Asn
    1010                1015                1020

Thr Asp Ala Ser Thr Thr Glu Glu Ser Thr Thr Ser Ala Gly Ser
    1025                1030                1035

Ser Thr Glu Gly Tyr Ser Ser Ser Ser His Asp Gly Ser Val Thr
    1040                1045                1050

Ser Thr Asp Gly Ser Ser Thr Ser Gly Gly Ala Ser Ser Ser Ser
    1055                1060                1065

Ala Ser Thr Ala Lys Ser Asp Ala Ala Ser Ser Glu Asp Gly Phe
    1070                1075                1080

Trp Trp Trp Asn Arg Arg Lys Ser Gly Ser Gly His Lys Ser Ala
    1085                1090                1095

Thr Val Gln Ser Ser Thr Thr Asp Lys Thr Ser Thr Asp Ser Ala
    1100                1105                1110

Ser Ser Thr Asp Ser Thr Ser Ser Thr Ser Gly Ala Ser Thr Thr
    1115                1120                1125

Thr Ser Gly Ser Ser Ser Thr Ser Gly Gly Ser Ser Thr Ser Asp
    1130                1135                1140

Ala Ser Ser Thr Ser Ser Ser Val Ser Arg Ser His Arg Ser Gly
    1145                1150                1155

Val Asn Arg Leu Leu His Lys Pro Gly Gln Gly Lys Ile Cys Leu
    1160                1165                1170

Cys Phe Glu Asn Ile Phe Asp Ile Pro Tyr His Leu Arg Lys Asn
    1175                1180                1185

Ile Gly Val
    1190

<210>  7
<211>  1186
<212>  PRT
<213>  Bombyx mori

<400>  7

Met Arg Phe Val Leu Cys Cys Thr Leu Ile Ala Leu Ala Ala Leu Ser
1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr
                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Glu Lys Ser
    450                 455                 460

Ala Ser Gln Ser Ser Ser Ser Arg Ser Ser Gln Glu Ser Ala Ser Tyr
465                 470                 475                 480

Ser Ser Ser Ser Ser Ser Ser Thr Leu Ser Glu Asp Ser Ser Glu Val
                485                 490                 495

Asp Ile Asp Leu Gly Asn Leu Gly Trp Trp Trp Asn Ser Asp Asn Lys
            500                 505                 510

Ala Gln Arg Ala Ala Gly Gly Ala Thr Lys Ser Glu Ala Ser Ser Ser
        515                 520                 525

Thr Gln Ala Thr Thr Val Ser Gly Ala Asp Asp Ser Ala Asp Ser Tyr
    530                 535                 540

Thr Trp Trp Trp Asn Pro Arg Arg Ser Ser Ser Ser Ser Ser Ser Ala
545                 550                 555                 560

Ser Ser Ser Ser Ser Gly Ser Asn Val Gly Gly Ser Ser Gln Ser Ser
                565                 570                 575

Gly Ser Ser Thr Ser Gly Ser Asn Ala Arg Gly His Leu Gly Thr Val
            580                 585                 590

Ser Ser Thr Gly Ser Thr Ser Asn Thr Asp Ser Ser Ser Lys Ser Ala
        595                 600                 605

Gly Ser Arg Thr Ser Gly Gly Ser Ser Thr Tyr Gly Tyr Ser Ser Ser
    610                 615                 620

His Arg Gly Gly Ser Val Ser Ser Thr Gly Ser Ser Ser Asn Thr Asp
625                 630                 635                 640

Ser Ser Thr Lys Asn Ala Gly Ser Ser Thr Ser Gly Gly Ser Ser Thr
                645                 650                 655

Tyr Gly Tyr Ser Ser Ser His Arg Gly Gly Ser Val Ser Ser Thr Gly
            660                 665                 670

Ser Ser Ser Asn Thr Asp Ser Ser Thr Lys Asn Ala Gly Ser Ser Thr
        675                 680                 685

Ser Gly Gly Ser Ser Thr Tyr Gly Tyr Ser Ser Ser His Arg Gly Gly
    690                 695                 700

Ser Val Ser Ser Thr Gly Ser Ser Ser Asn Thr Asp Ser Ser Thr Lys
705                 710                 715                 720

Ser Ala Gly Ser Ser Thr Ser Gly Gly Ser Ser Thr Tyr Gly Tyr Ser
                725                 730                 735

Ser Arg His Arg Gly Gly Arg Val Ser Ser Thr Gly Ser Ser Ser Thr
            740                 745                 750

Thr Asp Ala Ser Ser Asn Ser Val Gly Ser Ser Thr Ser Gly Gly Ser
        755                 760                 765

Ser Thr Tyr Gly Tyr Ser Ser Asn Ser Arg Asp Gly Ser Val Ser Ser
    770                 775                 780

Thr Gly Ser Ser Ser Asn Thr Asp Ser Asn Ser Asn Ser Ala Gly Ser
785                 790                 795                 800

Ser Thr Ser Gly Gly Ser Ser Thr Tyr Gly Tyr Ser Ser Asn Ser Arg
                805                 810                 815

Asp Gly Ser Val Ser Ser Thr Gly Ser Ser Ser Asn Thr Asp Ser Asn
            820                 825                 830

Ser Asn Ser Ala Gly Ser Ser Thr Ser Gly Gly Ser Ser Thr Tyr Gly
        835                 840                 845

Tyr Ser Ser Asn Ser Arg Asp Gly Ser Val Ser Ser Thr Gly Ser Ser
    850                 855                 860

Ser Asn Thr Asp Ala Ser Thr Asp Leu Thr Gly Ser Ser Thr Ser Gly
865                 870                 875                 880

Gly Ser Ser Thr Tyr Gly Tyr Ser Ser Asp Ser Arg Asp Gly Ser Val
                885                 890                 895

Ser Ser Thr Gly Ser Ser Ser Asn Thr Asp Ala Ser Thr Asp Leu Ala
            900                 905                 910

Gly Ser Ser Thr Ser Gly Gly Ser Ser Thr Tyr Gly Tyr Ser Ser Asp
        915                 920                 925

Cys Gly Asp Gly Ser Val Ser Ser Thr Gly Ser Ser Ser Asn Thr Asp
    930                 935                 940

Ala Ser Thr Asp Leu Ala Gly Ser Ser Thr Ser Gly Gly Ser Ser Thr
945                 950                 955                 960

Tyr Gly Tyr Ser Ser Asn Ser Arg Asp Gly Ser Val Ser Ser Thr Gly
                965                 970                 975

Ser Ser Ser Asn Thr Asp Ala Ser Thr Asp Leu Thr Gly Ser Ser Thr

            980                 985                 990

Ser Gly Gly Ser Ser Thr Tyr Gly Tyr Ser Ser Ser Asn Arg Asp Gly
        995                 1000                1005

Ser Val Leu Ala Thr Gly Ser Ser Ser Asn Thr Asp Ala Ser Thr
    1010                1015                1020

Thr Glu Glu Ser Thr Thr Ser Ala Gly Ser Ser Thr Glu Gly Tyr
    1025                1030                1035

Ser Ser Ser Ser His Asp Gly Ser Val Thr Ser Thr Asp Gly Ser
    1040                1045                1050

Ser Thr Ser Gly Gly Ala Ser Ser Ser Ser Ala Ser Thr Ala Lys
    1055                1060                1065

Ser Asp Ala Ala Ser Ser Glu Asp Gly Phe Trp Trp Trp Asn Arg
    1070                1075                1080

Arg Lys Ser Gly Ser Gly His Lys Ser Ala Thr Val Gln Ser Ser
    1085                1090                1095

Thr Thr Asp Lys Thr Ser Thr Asp Ser Ala Ser Ser Thr Asp Ser
    1100                1105                1110

Thr Ser Ser Thr Ser Gly Ala Ser Thr Thr Thr Ser Gly Ser Ser
    1115                1120                1125

Ser Thr Ser Gly Gly Ser Ser Thr Ser Asp Ala Ser Ser Thr Ser
    1130                1135                1140

Ser Ser Val Ser Arg Ser His Arg Ser Gly Val Asn Arg Leu Leu
    1145                1150                1155

His Lys Pro Gly Gln Gly Lys Ile Cys Leu Cys Phe Glu Asn Ile
    1160                1165                1170

Phe Asp Ile Pro Tyr His Leu Arg Lys Asn Ile Gly Val
    1175                1180                1185

<210> 8
<211> 1160
<212> PRT
<213> Bombyx mori

<400> 8

Met Arg Phe Val Leu Cys Cys Thr Leu Ile Ala Leu Ala Ala Leu Ser

1               5                   10                  15

Val Lys Ala Phe Gly His His Pro Gly Asn Arg Asp Thr Val Glu Val
            20                  25                  30

Lys Asn Arg Lys Tyr Asn Ala Ala Ser Ser Glu Ser Ser Tyr Leu Asn
        35                  40                  45

Lys Asp Asn Asp Ser Ile Ser Ala Gly Ala His Arg Ala Lys Ser Val
    50                  55                  60

Glu Gln Ser Gln Asp Lys Ser Lys Tyr Thr Ser Gly Pro Glu Gly Val
65                  70                  75                  80

Ser Tyr Ser Gly Arg Ser Gln Asn Tyr Lys Asp Ser Lys Gln Ala Tyr
                85                  90                  95

Ala Asp Tyr His Ser Asp Pro Asn Gly Gly Ser Ala Ser Ala Gly Gln
            100                 105                 110

Ser Arg Asp Ser Ser Leu Arg Glu Arg Asn Val His Tyr Val Ser Asp
        115                 120                 125

Gly Glu Ala Val Ala Ala Ser Ser Asp Ala Arg Asp Glu Asn Arg Ser
    130                 135                 140

Ala Gln Gln Asn Ala Gln Ala Asn Trp Asn Ala Asp Gly Ser Tyr Gly
145                 150                 155                 160

Val Ser Ala Asp Arg Ser Gly Ser Ala Ser Ser Arg Arg Arg Gln Ala
                165                 170                 175

Asn Tyr Tyr Ser Asp Lys Asp Ile Thr Ala Ala Ser Lys Asp Asp Ser
            180                 185                 190

Arg Ala Asp Ser Ser Arg Arg Ser Asn Ala Tyr Tyr Asn Arg Asp Ser
        195                 200                 205

Asp Gly Ser Glu Ser Ala Gly Leu Ser Asp Arg Arg Ala Ser Ser Ser
    210                 215                 220

Lys Asn Asp Asn Val Phe Val Tyr Arg Thr Lys Asp Ser Ile Gly Gly
225                 230                 235                 240

Gln Ala Lys Ser Ser Arg Ser Ser His Ser Gln Glu Ser Asp Ala Tyr
                245                 250                 255

Tyr Asn Ser Ser Pro Asp Gly Ser Tyr Asn Ala Gly Thr Arg Asp Ser
            260                 265                 270

Ser Ile Ser Asn Lys Lys Lys Ala Ser Ser Thr Ile Tyr Ala Asp Lys
        275                 280                 285

Asp Gln Ile Arg Ala Ala Asn Asp Arg Tyr Ser Ser Lys Gln Leu Lys
    290                 295                 300

Gln Ser Ser Ala Gln Ile Ser Ser Gly Pro Glu Gly Thr Ser Val Ser
305                 310                 315                 320

Ser Lys Asp Arg Gln Tyr Ser Asn Asp Lys Arg Ser Lys Ser Asp Ala
                325                 330                 335

Tyr Val Gly Arg Asp Gly Thr Val Ala Tyr Ser Asn Lys Asp Ser Glu
            340                 345                 350

Lys Thr Ser Arg Gln Ser Asn Thr Asn Tyr Ala Asp Gln Asn Ser Val
        355                 360                 365

Arg Ser Asp Ser Ala Ala Ser Asp Gln Thr Ser Asn Ser Tyr Asp Lys
    370                 375                 380

Gly Tyr Ser Asp Lys Asn Thr Val Ala His Ser Ser Gly Ser Arg Gly
385                 390                 395                 400

Ser Gln Asn Gln Lys Ser Ser Ser Tyr Arg Ala Asp Lys Asp Gly Phe
                405                 410                 415

Ser Ser Ser Thr Asn Thr Glu Lys Ser Arg Phe Ser Ser Ser Asn Ser
            420                 425                 430

Val Val Glu Thr Ser Asp Gly Thr Ser Ala Ser Arg Glu Ser Ser Ala
        435                 440                 445

Glu Asp Thr Lys Ser Ser Asn Ser Asn Val Gln Ser Asp Ala Leu Ser
    450                 455                 460

Glu Asp Ser Ser Glu Val Asp Ile Asp Leu Gly Asn Leu Gly Trp Trp
465                 470                 475                 480

Trp Asn Ser Asp Asn Lys Ala Gln Arg Ala Ala Gly Gly Ala Thr Lys
                485                 490                 495

Ser Glu Ala Ser Ser Ser Thr Gln Ala Thr Thr Val Ser Gly Ala Asp
            500                 505                 510

Asp Ser Ala Asp Ser Tyr Thr Trp Trp Trp Asn Pro Arg Arg Ser Ser
        515                 520                 525

Ser Ser Ser Ser Ser Ala Ser Ser Ser Ser Ser Gly Ser Asn Val Gly
    530                 535                 540

Gly Ser Ser Gln Ser Ser Gly Ser Ser Thr Ser Gly Ser Asn Ala Arg
545                 550                 555                 560

Gly His Leu Gly Thr Val Ser Ser Thr Gly Ser Thr Ser Asn Thr Asp
                565                 570                 575

Ser Ser Ser Lys Ser Ala Gly Ser Arg Thr Ser Gly Gly Ser Ser Thr
            580                 585                 590

Tyr Gly Tyr Ser Ser Ser His Arg Gly Gly Ser Val Ser Ser Thr Gly
        595                 600                 605

Ser Ser Ser Asn Thr Asp Ser Ser Thr Lys Asn Ala Gly Ser Ser Thr
    610                 615                 620

Ser Gly Gly Ser Ser Thr Tyr Gly Tyr Ser Ser Ser His Arg Gly Gly
625                 630                 635                 640

Ser Val Ser Ser Thr Gly Ser Ser Ser Asn Thr Asp Ser Ser Thr Lys
                645                 650                 655

Asn Ala Gly Ser Ser Thr Ser Gly Gly Ser Ser Thr Tyr Gly Tyr Ser
            660                 665                 670

Ser Ser His Arg Gly Gly Ser Val Ser Ser Thr Gly Ser Ser Ser Asn
        675                 680                 685

Thr Asp Ser Ser Thr Lys Ser Ala Gly Ser Ser Thr Ser Gly Gly Ser
    690                 695                 700

Ser Thr Tyr Gly Tyr Ser Ser Arg His Arg Gly Gly Arg Val Ser Ser
705                 710                 715                 720

Thr Gly Ser Ser Ser Thr Thr Asp Ala Ser Ser Asn Ser Val Gly Ser
                725                 730                 735

Ser Thr Ser Gly Gly Ser Ser Thr Tyr Gly Tyr Ser Ser Asn Ser Arg
            740                 745                 750

Asp Gly Ser Val Ser Ser Thr Gly Ser Ser Ser Asn Thr Asp Ser Asn
        755                 760                 765

Ser Asn Ser Ala Gly Ser Ser Thr Ser Gly Gly Ser Ser Thr Tyr Gly
    770                 775                 780

Tyr Ser Ser Asn Ser Arg Asp Gly Ser Val Ser Ser Thr Gly Ser Ser
785                 790                 795                 800

Ser Asn Thr Asp Ser Asn Ser Asn Ser Ala Gly Ser Ser Thr Ser Gly
                805                 810                 815

Gly Ser Ser Thr Tyr Gly Tyr Ser Ser Asn Ser Arg Asp Gly Ser Val
            820                 825                 830

Ser Ser Thr Gly Ser Ser Ser Asn Thr Asp Ala Ser Thr Asp Leu Thr
        835                 840                 845

Gly Ser Ser Thr Ser Gly Gly Ser Ser Thr Tyr Gly Tyr Ser Ser Asp
    850                 855                 860

Ser Arg Asp Gly Ser Val Ser Ser Thr Gly Ser Ser Ser Asn Thr Asp
865                 870                 875                 880

Ala Ser Thr Asp Leu Ala Gly Ser Ser Thr Ser Gly Gly Ser Ser Thr
                885                 890                 895

Tyr Gly Tyr Ser Ser Asp Cys Gly Asp Gly Ser Val Ser Ser Thr Gly
            900                 905                 910

Ser Ser Ser Asn Thr Asp Ala Ser Thr Asp Leu Ala Gly Ser Ser Thr
        915                 920                 925

Ser Gly Gly Ser Ser Thr Tyr Gly Tyr Ser Ser Asn Ser Arg Asp Gly
    930                 935                 940

Ser Val Ser Ser Thr Gly Ser Ser Ser Asn Thr Asp Ala Ser Thr Asp
945                 950                 955                 960

Leu Thr Gly Ser Ser Thr Ser Gly Gly Ser Ser Thr Tyr Gly Tyr Ser
                965                 970                 975

Ser Ser Asn Arg Asp Gly Ser Val Leu Ala Thr Gly Ser Ser Ser Asn
            980                 985                 990

Thr Asp Ala Ser Thr Thr Glu Glu  Ser Thr Thr Ser Ala  Gly Ser Ser
        995                 1000                1005

Thr Glu  Gly Tyr Ser Ser Ser  Ser His Asp Gly Ser  Val Thr Ser

1010                    1015                    1020

Thr Asp  Gly Ser Ser Thr Ser  Gly Gly Ala Ser Ser  Ser Ser Ala
    1025                    1030                    1035

Ser Thr  Ala Lys Ser Asp Ala  Ala Ser Ser Glu Asp  Gly Phe Trp
    1040                    1045                    1050

Trp Trp  Asn Arg Arg Lys Ser  Gly Ser Gly His Lys  Ser Ala Thr
    1055                    1060                    1065

Val Gln  Ser Ser Thr Thr Asp  Lys Thr Ser Thr Asp  Ser Ala Ser
    1070                    1075                    1080

Ser Thr  Asp Ser Thr Ser Ser  Thr Ser Gly Ala Ser  Thr Thr Thr
    1085                    1090                    1095

Ser Gly  Ser Ser Ser Thr Ser  Gly Gly Ser Ser Thr  Ser Asp Ala
    1100                    1105                    1110

Ser Ser  Thr Ser Ser Ser Val  Ser Arg Ser His Arg  Ser Gly Val
    1115                    1120                    1125

Asn Arg  Leu Leu His Lys Pro  Gly Gln Gly Lys Ile  Cys Leu Cys
    1130                    1135                    1140

Phe Glu  Asn Ile Phe Asp Ile  Pro Tyr His Leu Arg  Lys Asn Ile
    1145                    1150                    1155

Gly Val
    1160

<210>  9
<211>  779
<212>  PRT
<213>  Bombyx mori

<400>  9

Met Arg Phe Val Leu Cys Cys Thr Leu Ile Ala Leu Ala Ala Leu Ser
1               5                   10                  15

Val Lys Ala Phe Gly His His Gln Thr Gly Glu Glu Glu Leu Phe
            20                  25                  30

Asp Val Val Ser Tyr Gln Lys Ile Glu Asp Gly Lys Pro Val Ile Ile
        35                  40                  45

Met Lys Val Ile Pro Val Glu Lys Ser Ala Ser Gln Ser Ser Ser Ser

50                  55                  60

Arg Ser Ser Gln Glu Ser Ala Ser Tyr Ser Ser Ser Ser Ser Ser Ser
65                  70                  75                  80

Thr Leu Ser Glu Asp Ser Ser Glu Val Asp Ile Asp Leu Gly Asn Leu
                85                  90                  95

Gly Trp Trp Trp Asn Ser Asp Asn Lys Val Gln Arg Ala Ala Gly Gly
            100                 105                 110

Ala Thr Lys Ser Gly Ala Ser Ser Ser Thr Gln Ala Thr Thr Val Ser
        115                 120                 125

Gly Ala Asp Asp Ser Ala Asp Ser Tyr Thr Trp Trp Trp Asn Pro Arg
    130                 135                 140

Arg Ser Ser Ser Ser Ser Ser Ser Ala Ser Ser Ser Ser Ser Gly Ser
145                 150                 155                 160

Asn Val Gly Gly Ser Ser Gln Ser Ser Gly Ser Ser Thr Ser Gly Ser
                165                 170                 175

Asn Ala Arg Gly His Leu Gly Thr Val Ser Ser Thr Gly Ser Thr Ser
            180                 185                 190

Asn Thr Asp Ser Ser Ser Lys Ser Ala Gly Ser Arg Thr Ser Gly Gly
        195                 200                 205

Ser Ser Thr Tyr Gly Tyr Ser Ser Ser His Arg Gly Gly Ser Val Ser
    210                 215                 220

Ser Thr Gly Ser Ser Ser Asn Thr Asp Ser Ser Thr Lys Asn Ala Gly
225                 230                 235                 240

Ser Ser Thr Ser Gly Gly Ser Ser Thr Tyr Gly Tyr Ser Ser Ser His
                245                 250                 255

Arg Gly Gly Ser Val Ser Ser Thr Gly Ser Ser Ser Asn Thr Asp Ser
            260                 265                 270

Ser Thr Lys Ser Ala Gly Ser Ser Thr Ser Gly Gly Ser Ser Thr Tyr
        275                 280                 285

Gly Tyr Ser Ser Arg His Arg Gly Gly Arg Val Ser Ser Thr Gly Ser
    290                 295                 300

Ser Ser Thr Thr Asp Ala Ser Ser Asn Ser Val Gly Ser Ser Thr Ser
305                 310                 315                 320

Gly Gly Ser Ser Thr Tyr Gly Tyr Ser Ser Asn Ser Arg Asp Gly Ser
                325                 330                 335

Val Ser Ser Thr Gly Ser Ser Ser Asn Thr Asp Ser Asn Ser Asn Ser
            340                 345                 350

Ala Gly Ser Ser Thr Ser Gly Gly Ser Ser Thr Tyr Gly Tyr Ser Ser
        355                 360                 365

Asn Ser Arg Asp Gly Ser Val Ser Ser Thr Gly Ser Ser Ser Asn Thr
    370                 375                 380

Asp Ser Asn Ser Asn Ser Ala Gly Ser Ser Thr Ser Gly Gly Ser Ser
385                 390                 395                 400

Thr Tyr Gly Tyr Ser Ser Asn Ser Arg Asp Gly Ser Val Ser Ser Thr
                405                 410                 415

Gly Ser Ser Ser Asn Thr Asp Ala Ser Thr Asp Leu Thr Gly Ser Ser
            420                 425                 430

Thr Ser Gly Gly Ser Ser Thr Tyr Gly Tyr Ser Ser Asp Ser Arg Asp
        435                 440                 445

Gly Ser Val Ser Ser Thr Gly Ser Ser Ser Asn Thr Asp Ala Ser Thr
    450                 455                 460

Asp Leu Ala Gly Ser Ser Thr Ser Gly Gly Ser Ser Thr Tyr Gly Tyr
465                 470                 475                 480

Ser Ser Asp Cys Gly Asp Gly Ser Val Ser Ser Thr Gly Ser Ser Ser
                485                 490                 495

Asn Thr Asp Ala Ser Thr Asp Leu Ala Gly Ser Ser Thr Ser Gly Gly
            500                 505                 510

Ser Ser Thr Tyr Gly Tyr Ser Ser Asp Ser Arg Asp Gly Ser Val Ser
        515                 520                 525

Ser Thr Gly Ser Ser Ser Asn Thr Asp Ala Ser Thr Asp Leu Ala Gly
    530                 535                 540

Ser Ser Thr Ser Gly Gly Ser Ser Thr Tyr Gly Tyr Ser Ser Asn Ser
545                 550                 555                 560

Arg Asp Gly Ser Val Ser Ser Thr Gly Ser Ser Ser Asn Thr Asp Ala
                565                 570                 575

Ser Thr Asp Leu Thr Gly Ser Ser Thr Ser Gly Gly Ser Ser Thr Tyr
            580                 585                 590

Gly Tyr Ser Ser Ser Asn Arg Asp Gly Ser Val Leu Ala Thr Gly Ser
        595                 600                 605

Ser Ser Asn Thr Asp Ala Ser Thr Thr Glu Glu Ser Thr Thr Ser Ala
    610                 615                 620

Gly Ser Ser Thr Glu Gly Tyr Ser Ser Ser Ser His Asp Gly Ser Val
625                 630                 635                 640

Thr Ser Thr Asp Gly Ser Ser Thr Ser Gly Gly Ala Ser Ser Ser Ser
                645                 650                 655

Ala Ser Thr Ala Lys Ser Asp Ala Ala Ser Ser Glu Asp Gly Phe Trp
            660                 665                 670

Trp Trp Asn Arg Arg Lys Ser Gly Ser Gly His Lys Ser Ala Thr Val
        675                 680                 685

Gln Ser Ser Thr Thr Asp Lys Thr Ser Thr Asp Ser Ala Ser Ser Thr
    690                 695                 700

Asp Ser Thr Ser Ser Thr Ser Gly Ala Ser Thr Thr Thr Ser Gly Ser
705                 710                 715                 720

Ser Ser Thr Ser Gly Gly Ser Ser Thr Ser Asp Ala Ser Ser Thr Ser
                725                 730                 735

Ser Ser Val Ser Arg Ser His His Ser Gly Val Asn Arg Leu Leu His
            740                 745                 750

Lys Pro Gly Gln Gly Lys Ile Cys Leu Cys Phe Glu Asn Ile Phe Asp
        755                 760                 765

Ile Pro Tyr His Leu Arg Lys Asn Ile Gly Val
    770                 775

<210>  10
<211>  722
<212>  PRT
<213>  Bombyx mori

<400>  10

Met Arg Phe Val Leu Cys Cys Thr Leu Ile Ala Leu Ala Ala Leu Ser
1               5                   10                  15

Val Lys Ala Phe Gly His His Pro Leu Ser Glu Asp Ser Ser Glu Val
            20                  25                  30

Asp Ile Asp Leu Gly Asn Leu Gly Trp Trp Trp Asn Ser Asp Asn Lys
        35                  40                  45

Ala Gln Arg Ala Ala Gly Gly Ala Thr Lys Ser Glu Ala Ser Ser Ser
    50                  55                  60

Thr Gln Ala Thr Thr Val Ser Gly Ala Asp Asp Ser Ala Asp Ser Tyr
65                  70                  75                  80

Thr Trp Trp Trp Asn Pro Arg Arg Ser Ser Ser Ser Ser Ser Ser Ala
                85                  90                  95

Ser Ser Ser Ser Ser Gly Ser Asn Val Gly Gly Ser Ser Gln Ser Ser
            100                 105                 110

Gly Ser Ser Thr Ser Gly Ser Asn Ala Arg Gly His Leu Gly Thr Val
        115                 120                 125

Ser Ser Thr Gly Ser Thr Ser Asn Thr Asp Ser Ser Ser Lys Ser Ala
    130                 135                 140

Gly Ser Arg Thr Ser Gly Gly Ser Ser Thr Tyr Gly Tyr Ser Ser Ser
145                 150                 155                 160

His Arg Gly Gly Ser Val Ser Ser Thr Gly Ser Ser Ser Asn Thr Asp
                165                 170                 175

Ser Ser Thr Lys Asn Ala Gly Ser Ser Thr Ser Gly Gly Ser Ser Thr
            180                 185                 190

Tyr Gly Tyr Ser Ser Ser His Arg Gly Gly Ser Val Ser Ser Thr Gly
        195                 200                 205

Ser Ser Ser Asn Thr Asp Ser Ser Thr Lys Ser Ala Gly Ser Ser Thr
    210                 215                 220

Ser Gly Gly Ser Ser Thr Tyr Gly Tyr Ser Ser Arg His Arg Gly Gly
225                 230                 235                 240

Arg Val Ser Ser Thr Gly Ser Ser Ser Thr Thr Asp Ala Ser Ser Asn
                245                 250                 255

Ser Val Gly Ser Ser Thr Ser Gly Gly Ser Ser Thr Tyr Gly Tyr Ser
            260                 265                 270

Ser Asn Ser Arg Asp Gly Ser Val Ser Ser Thr Gly Ser Ser Ser Asn
        275                 280                 285

Thr Asp Ser Asn Ser Asn Ser Ala Gly Ser Ser Thr Ser Gly Gly Ser
    290                 295                 300

Ser Thr Tyr Gly Tyr Ser Ser Asn Ser Arg Asp Gly Ser Val Ser Ser
305                 310                 315                 320

Thr Gly Ser Ser Ser Asn Thr Asp Ser Asn Ser Asn Ser Ala Gly Ser
                325                 330                 335

Ser Thr Ser Gly Gly Ser Ser Thr Tyr Gly Tyr Ser Ser Asn Ser Arg
            340                 345                 350

Asp Gly Ser Val Ser Ser Thr Gly Ser Ser Ser Asn Thr Asp Ala Ser
        355                 360                 365

Thr Asp Leu Thr Gly Ser Ser Thr Ser Gly Gly Ser Ser Thr Tyr Gly
    370                 375                 380

Tyr Ser Ser Asp Ser Arg Asp Gly Ser Val Ser Ser Thr Gly Ser Ser
385                 390                 395                 400

Ser Asn Thr Asp Ala Ser Thr Asp Leu Ala Gly Ser Ser Thr Ser Gly
                405                 410                 415

Gly Ser Ser Thr Tyr Gly Tyr Ser Ser Asp Cys Gly Asp Gly Ser Val
            420                 425                 430

Ser Ser Thr Gly Ser Ser Ser Asn Thr Asp Ala Ser Thr Asp Leu Ala
        435                 440                 445

Gly Ser Ser Thr Ser Gly Gly Ser Ser Thr Tyr Gly Tyr Ser Ser Asp
    450                 455                 460

Ser Arg Asp Gly Ser Val Ser Ser Thr Gly Ser Ser Ser Asn Thr Asp
465                 470                 475                 480

Ala Ser Thr Asp Leu Ala Gly Ser Ser Thr Ser Gly Gly Ser Ser Thr
                485                 490                 495

Tyr Gly Tyr Ser Ser Asn Ser Arg Asp Gly Ser Val Ser Ser Thr Gly

            500                 505                 510

Ser Ser Ser Asn Thr Asp Ala Ser Thr Asp Leu Thr Gly Ser Ser Thr
        515                 520                 525

Ser Gly Gly Ser Ser Thr Tyr Gly Tyr Ser Ser Ser Asn Arg Asp Gly
    530                 535                 540

Ser Val Ser Ala Thr Gly Ser Ser Ser Asn Thr Asp Ala Ser Thr Thr
545                 550                 555                 560

Glu Glu Ser Thr Thr Ser Ala Gly Ser Ser Thr Glu Gly Tyr Ser Ser
                565                 570                 575

Ser Ser His Asp Gly Ser Val Thr Ser Thr Asp Gly Ser Ser Thr Ser
            580                 585                 590

Gly Gly Ala Ser Ser Ser Ser Ala Ser Thr Ala Lys Ser Asp Ala Ala
        595                 600                 605

Ser Ser Glu Asp Gly Phe Trp Trp Trp Asn Arg Arg Lys Ser Gly Ser
    610                 615                 620

Gly His Lys Ser Ala Thr Val Gln Ser Ser Thr Thr Asp Lys Thr Ser
625                 630                 635                 640

Thr Asp Ser Ala Ser Ser Thr Asp Ser Thr Ser Ser Thr Ser Gly Ala
                645                 650                 655

Ser Thr Thr Thr Ser Gly Ser Ser Ser Thr Ser Gly Gly Ser Ser Thr
            660                 665                 670

Ser Asp Ala Ser Ser Thr Ser Ser Ser Val Ser Arg Ser His His Ser
        675                 680                 685

Gly Val Asn Arg Leu Leu His Lys Pro Gly Gln Gly Lys Ile Cys Leu
    690                 695                 700

Cys Phe Glu Asn Ile Phe Asp Ile Pro Tyr His Leu Arg Lys Asn Ile
705                 710                 715                 720

Gly Val